# EUROPÄISCHE PATENTSCHRIFT

(11) **EP 1 802 561 B1**
(45) Veröffentlichungstag und Bekanntmachung des Hinweises auf die Patenterteilung: **12.01.2011**
(21) Anmeldenummer: 05805158.2
(22) Anmeldetag: 08.10.2005
(51) Int. Cl.: C07C 45/62, C07C 47/21, C07C 29/17, C07C 35/12

(54) **VERFAHREN ZUR HERSTELLUNG OPTISCH AKTIVER CARBONYLVERBINDUNGEN**
METHOD FOR THE PRODUCTION OF OPTICALLY ACTIVE CARBONYL COMPOUNDS
PROCEDE POUR PREPARER DES COMPOSES DE CARBONYLE OPTIQUEMENT ACTIFS

(30) Priorität: 11.10.2004 DE 102004049631
(43) Veröffentlichungstag der Anmeldung: 04.07.2007
(73) Patentinhaber: BASF SE, 67056 Ludwigshafen (DE)
(72) Erfinder: JÄKEL, Christoph, 67117 Limburgerhof (DE); PACIELLO, Rocco, 67098 Bad Dürkheim (DE)
(86) Internationale Anmeldenummer: PCT/EP2005/010847
(87) Internationale Veröffentlichungsnummer: WO 2006/040096

(56) Entgegenhaltungen:
- EP-A- 0 000 315
- EP-A- 1 053 974
- US-A- 4 237 072
- DATABASE WPI Section Ch, Week 197733 Derwent Publications Ltd., London, GB; Class E14, AN 1977-58267Y XP002364613 & JP 52 078812 A (NIKKI CHEM CO LTD) 2. Juli 1977 (1977-07-02)

## Beschreibung

Die vorliegende Erfindung betrifft ein Verfahren zur Herstellung optisch aktiver Carbonylverbindungen durch asymmetrische Hydrierung α,β-ungesättigter Carbonylverbindungen in Gegenwart von im Reaktionsgemisch löslicher, optisch aktiver Übergangsmetall-Katalysatoren, die mindestens einen Kohlenmonoxid-Liganden aufweisen. Speziell betrifft die vorliegende Erfindung ein Verfahren zur Herstellung optisch aktiver Aldehyde oder Ketone, insbesondere Citronellal durch asymmetrische Hydrierung der entsprechenden optisch aktiven α,β-ungesättigten Aldehyde oder Ketone.

Viele optisch aktive Aldehyde und Ketone stellen wertvolle Zwischenstoffe zur Synthese höher veredelter chiraler Wert- und Wirkstoffe dar und sind ihrerseits oft begehrte Riech- und Aromastoffe.

Die EP-A 0 000 315 betrifft ein Verfahren zur Herstellung von optisch aktivem Citronellal durch Hydrierung von Geranial oder Neral in Gegenwart eines im Reaktionssystem gelösten Katalysatorkomplexes aus Rhodium und einem chiralen Phosphin.

T.-P. Dang et al. beschreiben in J. Mol. Cat. 1982, 16, 51 - 59 die homogenkatalytische Hydrierung α,β-ungesättigter Aldhyde sowie die Anwendung des Verfahrens zur Herstellung von optisch aktivem Citronellal. Als Katalysatoren dienten dabei Komplexverbindungen aus einem Rhodiumcarbonyl und einem chiralen Diphosphin.

Auch Chapuis et al. erwähnen in Hev. Chim. Acta 2001, 84, 230 -242, Fußnote 4, die asymmetrische Hydrierung von Geranial bzw. Neral zu optisch aktivem Citronellal in Gegenwart eines Katalysators aus Rh₄(CO)₁₂ und (R,R)-Chiraphos (2R, 3R)-2,3-bis(diphenylphosphino)butan.

Ein Problem bei der Durchführung mittels löslicher Katalysatoren katalysierter (homogenkatalytischer) Reaktionen besteht in der oft unzureichenden Stabilität der eingesetzten Katalysatorkomplexe bzw. der sich daraus bildenden katalytisch aktiven Metall- bzw. Übergangsmetall-Komplexverbindung. Vor dem Hintergrund des oft hohen Preises derartiger Katalysatoren bzw. Katalysatorvorstufen sind homogenkatalytische Reaktionen mit komplexen Übergangsmetallkatalysatoren nur in speziellen Fällen in wirtschaftlicher Weise im technischen Maßstab anwendbar.

Die JP-A 52078812 beschreibt ein Verfahren zur Hydrierung α,β-ungesättigter Aldehyde wie Crotonaldehyd, Zimtaldehyd oder α-Methylzimtaldehyd an homogenen Rh-Katalysatoren unter Hydroformylierungsbedingungen in Gegenwart eines Triarylphosphins, eines tertiären Amins in stöchiometrischer Menge und Kohlenmonoxid. Aufgabe der vorliegenden Erfindung war es, ein Verfahren zur homogenkatalytischen asymmetrischen Hydrierung α,β-ungesättigter Aldehyde oder Ketone bereitzustellen, dass sich durch eine erhöhte Stabilität und damit durch erhöhte Lebensdauer der katalytisch aktiven, in optisch aktiver Form einzusetzenden Übergangsmetall-Komplexverbindung auszeichnet und sich dadurch für Anwendungen im technischen Maßstab in besonderem Maße eignet.

Die Aufgabe wurde überraschend gelöst durch Bereitstellung eines Verfahrens zur Herstellung optisch aktiver Carbonylverbindungen durch asymmetrische Hydrierung α,β-ungesättigter Carbonylverbindungen in Gegenwart von im Reaktionsgemisch löslichen, optisch aktiven Übergangsmetall-Katalysatoren, die mindestens einen Kohlenmonoxid-Liganden aufweisen, das dadurch gekennzeichnet ist, dass man den Katalysator mit einem Kohlenmonoxid und Wasserstoff enthaltenden Gasgemisch vorbehandelt und/oder die asymmetrische Hydrierung in Gegenwart von dem Reaktionsgemisch zusätzlich zugeführtem Kohlenmonoxid durchführt.

Das erfindungsgemäße Verfahren eignet sich zur Herstellung optisch aktiver Carbonylverbindungen wie Aldehyden, Ketonen, Estern, Lactonen oder Lactamen durch asymmetrische, d.h. enantioselektive Hydrierung der entsprechenden Carbonylverbindungen, die in α,β-Position zur Carbonylgruppe eine ethylenische Doppelbindung aufweisen. Erfindungsgemäß hydriert man die zur Carbonylgruppe α,β-ständige ethylenische Doppelbindung in Gegenwart eines im Reaktionsgemisch löslichen, optisch aktiven Übergangsmetallkatalysators, der mindestens einen Kohlenmonoxid- bzw. CO-Liganden aufweist, zu einer Kohlenstoff-Kohlenstoff-Einfachbindung, wobei das dadurch in β-Position neu geschaffene tetraedrische Kohlenstoffatom asymmetrisch substituiert ist und in nicht-racemischer Form erhalten wird. Unter dem Begriff asymmetrische Hydrierung ist demgemäss im Rahmen der vorliegenden Erfindung eine Hydrierung zu verstehen, bei der die beiden enantiomeren Formen des Hydrierproduktes nicht zu gleichen Teilen erhalten werden. Das erfindungsgemäße Verfahren ist dadurch gekennzeichnet, dass man den eingesetzten, im Reaktionsgemisch löslichen, d.h. homogenen Katalysator entweder vor der asymmetrischen Hydrierung mit einem Gasgemisch vorbehandelt, das Kohlenmonoxid und Wasserstoff enthält (d.h. eine sogenannte Präformierung durchführt) oder die asymmetrische Hydrierung in Gegenwart von dem Reaktionsgemisch zusätzlich zugeführtem Kohlenmonoxid durchführt oder eine Präformierung durchführt und anschließend die asymmetrische Hydrierung in Gegenwart von dem Reaktionsgemisch zusätzlich zugeführtem Kohlenmonoxid durchführt.

Die erfindungsgemäß einzusetzenden, im Reaktionsgemisch löslichen Übergangsmetall-Katalysatoren weisen zumindest in einer im Katalysezyklus durchlaufenen Form oder in einer dem eigentlichen Katalysezyklus vorgeschalteten Vorform mindestens einen CO-Liganden auf, wobei es unerheblich ist, ob diese mindestens einen CO-Liganden aufweisende Katalysatorform die tatsächliche katalytisch aktive Katalysatorform darstellt. Im Rahmen des erfindungsgemäßen Verfahrens stabilisiert man die mindestens einen CO-Liganden aufweisende Katalysatorform durch dem Reaktionsgemisch zusätzlich zugeführtem Kohlenmonoxid in vorteilhafter Weise. Dabei ist insbesondere überraschend, dass das als Katalysatorgift bekannte Kohlenmonoxid auch zur Unterstützung der erfindungsgemäß durchzuführenden Umsetzung eingesetzt werden kann.

Das erfindungsgemäße Verfahren eignet sich in besonderem Maße zur Herstellung optisch aktiver Carbonylverbindungen der Formel (I) 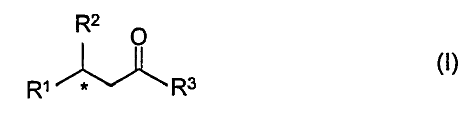 wobei die Reste
- R¹, R²: jeweils für einen unverzweigten, verzweigten oder cyclischen Alkylrest mit 1 bis 25 Kohlenstoffatomen, der gegebenenfalls eine oder mehrere, in der Re- gel 1 bis etwa 5, ethylenische Doppelbindungen und/oder einen oder mehre- re, in der Regel 1 bis etwa 5, gleiche oder verschiedene Substituenten aus- gewählt aus der Gruppe der Substituenten OR⁴, NR⁵R⁶, Halogen, C₆-C₁₀-Aryl und C₃-C₉-Hetaryl tragen kann und der gemeinsam mit R³ einen 5 bis 25- gliedrigen Ring bilden kann, steht, mit der Maßgabe, dass R¹ und R² ver- schieden sind,
- R³: für Wasserstoff oder einen unverzweigten, verzweigten oder cyclischen Al- kylrest mit 1 bis 25 Kohlenstoffatomen, der gegebenenfalls eine oder mehre- re, in der Regel 1 bis etwa 5, ethylenische Doppelbindungen und/oder einen oder mehrere, in der Regel 1 bis etwa 5, gleiche oder verschiedene Substi- tuenten ausgewählt aus der Gruppe der Substituenten OR⁴, NR⁵R⁶, Halogen, C₆-C₁₀-Aryl und C₃-C₉-Hetaryl tragen kann, oder für steht OR⁷ oder NR⁸R⁹ steht
wobei
- R⁴, R⁵, R⁶: jeweils unabhängig voneinander Wasserstoff, C₁-C₆-Alkyl, C₆-C₁₀- Aryl, C₇-C₁₂-Aralkyl oder C₇-C₁₂-Alkylaryl bedeuten und
- R⁵ und R⁶: gemeinsam auch eine Alkylenkette mit 2 bis 5 Kohlenstoffatomen, die durch N oder O unterbrochen sein kann, bedeuten können und
- R⁷, R⁸: für einen unverzweigten, verzweigten oder cyclischen Alkylrest mit 1 bis 25 Kohlenstoffatomen, der gegebenenfalls eine oder mehrere, in der Regel 1 bis etwa 5, ethylenische Doppelbindungen und/oder ei- nen oder mehrere, in der Regel 1 bis etwa 5, gleiche oder verschie- dene Substituenten ausgewählt aus der Gruppe der Substituenten OR⁴, NR⁵R⁶, Halogen, C₆-C₁₀-Aryl und C₃-C₉-Hetaryl tragen kann, steht und gemeinsam mit R¹ oder R² einen 5 bis 25-gliedrigen Ring bilden können und
- R⁹: für Wasserstoff C₁-C₆-Alkyl, C₆-C₁₀-Aryl, C₇-C₁₂-Aralkyl oder C₇-C₁₂- Alkylaryl steht und
- *: ein asymmetrisches Kohlenstoffatom bezeichnet,
durch asymmetrische Hydrierung α,β-ungesättigter Aldehyde oder Ketone der Formel (II) 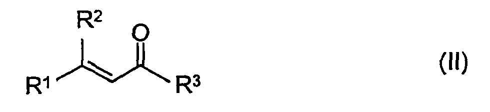 wobei die Reste R¹ bis R³ die oben angegebene Bedeutung besitzen.

Beispielhaft seien für die genannten Substituenten bzw. Reste folgende Bedeutungen im Rahmen der vorliegenden Erfindung angegeben:
C₁-C₆-Alkyl steht beispielsweise für Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl.
C₆-C₁₀-Aryl bedeutet beispielsweise Phenyl oder Naphtyl.
C₁-C₁₂-Aralkyl bedeutet beispielsweise Phenylmethyl, 1-Phenylethyl 2-Phenylethyl, 1-Phenylpropyl, 2-Phenylpropyl oder 3-Phenylpropyl.
C₃-C₉-Hetaryl steht beispielsweise für 2-Furyl, 3-Furyl, 2-Pyrroyl, 3-Pyrroyl, 2-Thienyl, 3-Thienyl, 2-Pyridyl, 3-Pyridyl, 4-Pyridyl, 2-Indolyl, 3-Indolyl, 4-Indolyl, 5-Indolyl, 6-Indolyl, 7-Indolyl.
C₇-C₁₂-Alkylaryl steht beispielsweise für 1-Methylphenyl, 2-Methylphenyl, 3-Methylphenyl, 1-Ethylphenyl, 2-Ethylphenyl, 3-Ethylphenyl, 1-Propylphenyl, 2-Propylphenyl, 3-Propylphenyl, 1-iso-Propylphenyl, 2-iso-Propylphenyl, 3-iso-Propylphenyl, 1-Butylphenyl, 2-Butylphenyl, 3-Butylphenyl, 1-iso-Butylphenyl, 2-iso-Butylphenyl, 3-iso-Butylphenyl, 1-sec-Butylphenyl, 2-sec-Butylphenyl, 3-sec-Butylphenyl, 1-tert-Butylphenyl, 2-tert-Butylphenyl, 3-tert-Butylphenyl, 1-(1-pentenyl)phenyl, 2-(1-pentenyl)phenyl, 3-(1-pentenyl)phenyl, 1-(2-pentenyl)phenyl, 2-(2-pentenyl)phenyl, 3-(2-pentenyl)phenyl, 1-(3-pentenyl)phenyl, 2-(3-pentenyl)phenyl, 3-(3-pentenyl)phenyl, 1-(1-(2-methylbutyl))phenyl, 2-(1-(2-methylbutyl))phenyl, 3-(1-(2-methylbutyl))phenyl, 1-(2-(2-methylbutyl))phenyl, 2-(2-(2-methylbutyl))phenyl, 3-(2-(2-methylbutyl))phenyl, 1-(3-(2-methylbutyl))phenyl, 2-(3-(2-methylbutyl))phenyl, 3-(3-(2-methylbutyl))phenyl, 1-(4-(2-methylbutyl))phenyl, 2-(4-(2-methylbutyl))phenyl, 3-(4-(2-methylbutyl))phenyl, 1-(1-(2,2-Dimethylpropyl))phenyl, 2-(1-(2,2-Dimethylpropyl))phenyl, 3-(1-(2,2-Dimethylpropyl))phenyl, 1-(1-hexenyl)phenyl, 2-(1-hexenyl)phenyl, 3-(1-hexenyl)phenyl, 1-(2-hexenyl)phenyl, 2-(2-hexenyl)phenyl, 3-(2-hexenyl)phenyl, 1-(3-hexenyl)phenyl, 2-(3-hexenyl)phenyl, 3-(3-hexenyl)phenyl, 1-(1-(2-Methylpentenyl))phenyl, 2-(1-(2-Methylpentenyl))phenyl, 3-(1-(2-Methylpentenyl))phenyl, 1-(2-(2-Methylpentenyl))phenyl, 2-(2-(2-Methylpentenyl))phenyl, 3-(2-(2-Methylpentenyl))phenyl, 1-(3-(2-Methylpentenyl))phenyl, 2-(3-(2-Methylpentenyl))phenyl, 3-(3-(2-Methylpentenyl))phenyl, 1-(4-(2-Methylpentenyl))phenyl, 2-(4-(2-Methylpentenyl))phenyl, 3-(4-(2-Methylpentenyl))phenyl, 1-(5-(2-Methylpentenyl))phenyl, 2-(5-(2-Methylpentenyl))phenyl, 3-(5-(2-Methylpentenyl))phenyl, 1-(1-(2,2-Dimethylbutenyl))phenyl, 2-(1-(2,2-Dimethylbutenyl))phenyl, 3-(1-(2,2-Dimethylbutenyl))phenyl, 1-(3-(2,2-Dimethylbutenyl))phenyl, 2-(3-(2,2-Dimethylbutenyl))phenyl, 3-(3-(2,2-Dimethylbutenyl))phenyl, 1-(4-(2,2-Dimethylbutenyl))phenyl, 2-(4-(2,2-Dimethylbutenyl))phenyl, 3-(4-(2,2-Dimethylbutenyl))phenyl.

Unter Halogen ist im Rahmen der vorliegenden Erfindung Fluor, Chlor, Brom oder lod, bevorzugt Fluor, Chlor oder Brom zu verstehen.

Das erfindungsgemäße Verfahren eignet sich demgemäss beispielsweise zur Herstellung der folgenden, beispielhaft genannten Verbindungen der Formeln (I-1) bis (I-25) in optisch aktiver Form:

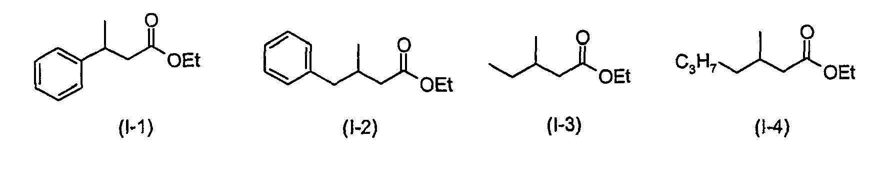

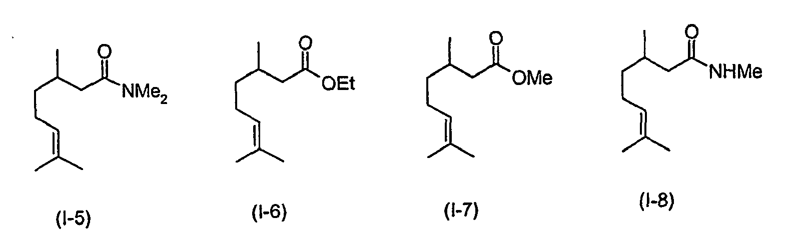

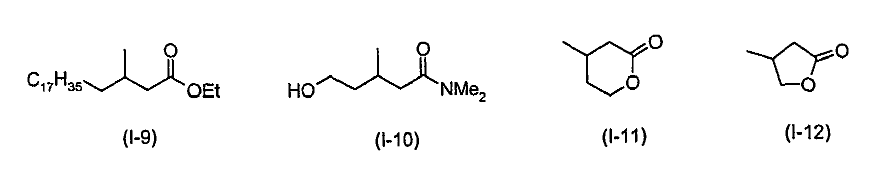

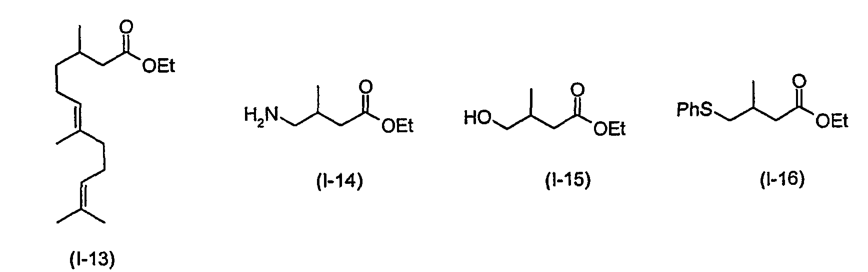

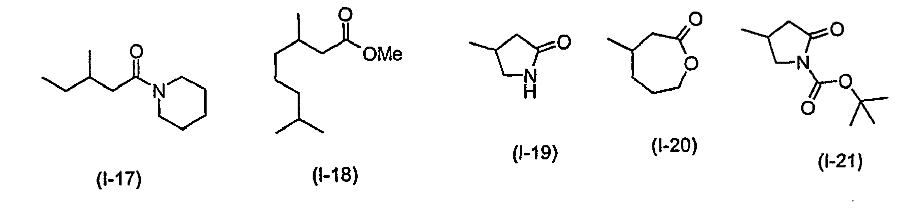

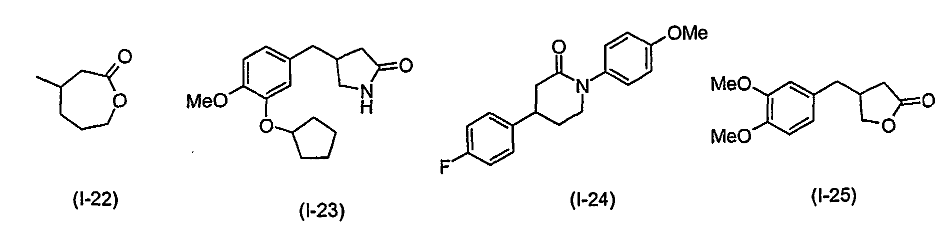

Insbesondere eignet sich das erfindungsgemäße Verfahren zur Herstellung optisch aktiver Aldehyde oder Ketone durch asymmetrische Hydrierung α,β-ungesättigter Aldehyde oder Ketone. Demgemäss eignet es sich insbesondere zur Herstellung optisch aktiver Verbindungen der Formel (I') 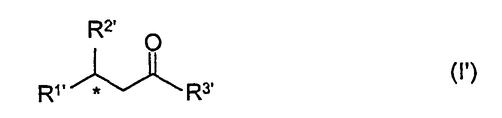 in der
- R^{1'}, R^{2'}: die gleichen Bedeutungen haben können wie vorstehend R¹ und R² und
- R³': für Wasserstoff oder einen unverzweigten, verzweigten oder cyclischen Alkylrest mit 1 bis 25 Kohlenstoffatomen, der gegebenenfalls eine oder mehrere, in der Regel 1 bis etwa 5, ethylenische Doppelbindungen und/oder einen oder mehrere, in der Regel 1 bis etwa 5, gleiche oder ver- schiedene Substituenten ausgewählt aus der Gruppe der Substituenten OR⁴, NR⁵R⁶, Halogen, C₆-C₁₀-Aryl und C₃-C₉-Hetaryl tragen kann, steht,
wobei R⁴, R⁵ und R⁶ die vorstehend bezeichneten Bedeutungen besitzen können,
durch asymmetrische Hydrierung α,β-ungesättigter Aldehyde oder Ketone der Formel (II') 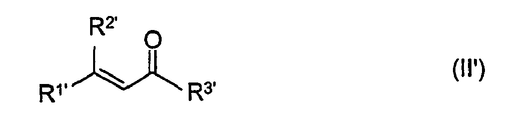 wobei die Reste R^{1'} bis R^{3'} die oben angegebene Bedeutung besitzen.

Bevorzugt eignet sich das erfindungsgemäße Verfahren zur Herstellung optisch aktiver Aldehyde der Formel (III), die in β-Position zur Carbonylgruppe eine Methylgruppe aufweisen 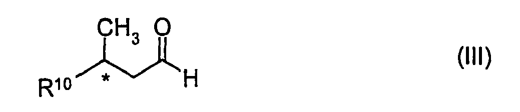 wobei
- R¹⁰: für einen unverzweigten oder verzweigten Alkylrest mit 2 bis 25 Kohlenstoff- atomen, der gegebenenfalls 1 bis 5 ethylenische Doppelbindungen aufweisen kann, steht und
- *: ein asymmetrisches Kohlenstoffatom bezeichnet,
durch asymmetrische Hydrierung α,β-ungesättigter Aldehyde der Formel (IV) oder (V) 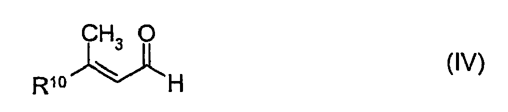 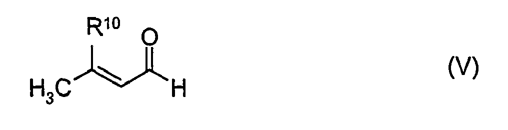 wobei der Rest R¹⁰ die oben angegebene Bedeutung besitzt.

Als Beispiele für erfindungsgemäß in optisch aktiver Form herstellbare Aldehyde oder Ketone der Formeln (I') bzw. (III) seien die folgenden Verbindungen genannt:

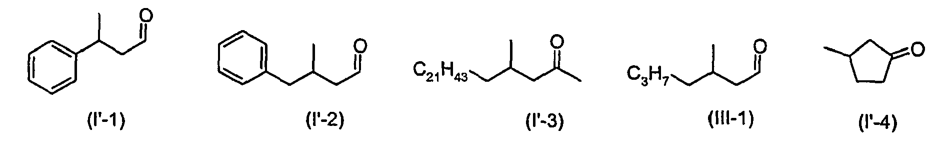

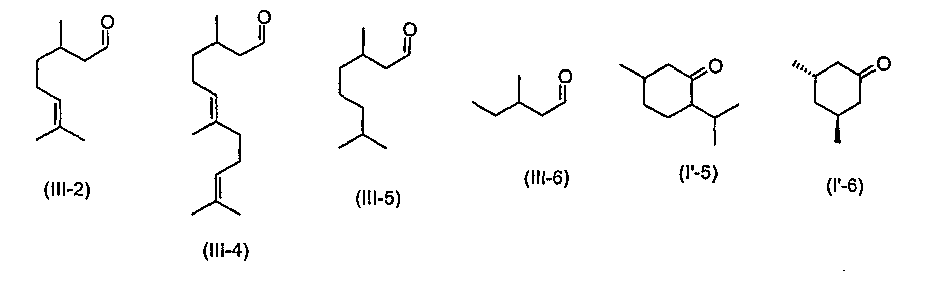

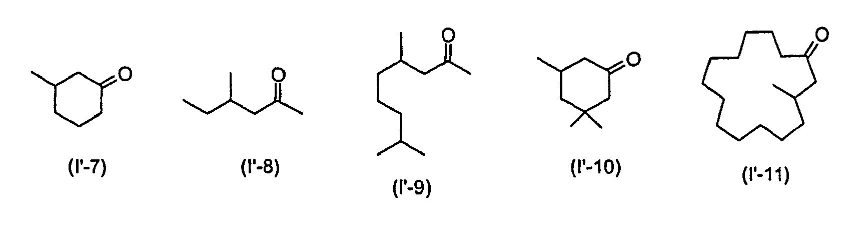

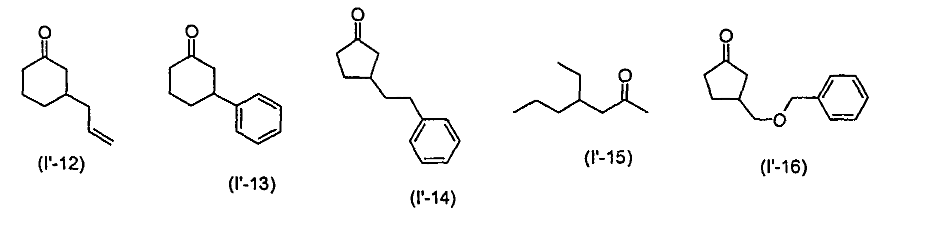

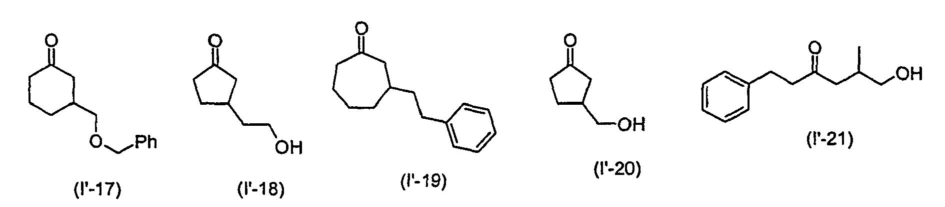

Die Aldehyde der Formel (III) sind erfindungsgemäß durch asymmetrische, d.h. enantioselektive Hydrierung der entsprechenden α,β-ungesättigten Aldehyde der Formeln (IV) oder (V) zugänglich. Die Verbindungen der Formeln (IV) und (V) stellen E/Z-Doppelbindungsisomere zueinander dar. Prinzipiell sind die optisch aktiven Aldehyde der Formel (III) ausgehend von beiden Doppelbindungsisomeren der Formeln (IV) und (V) zugänglich. Je nach Wahl der enantiomeren Form des Katalysators , d.h. je nach Wahl des (+)- bzw. (-)-Enantiomeren des Katalysators bzw. des (+)- bzw. (-)-Enantiomeren des eingesetzten chiralen Liganden, erhält man in erfindungsgemäßer Weise aus dem eingesetzten E- oder Z-Doppelbindungsisomeren bevorzugt eines der Enantiomere des optisch aktiven Aldehyds. Gleiches gilt für die vorstehend genannten Substrat bzw. Produktklassen. Prinzipiell lassen sich auch Gemische der beiden Doppelbindungsisomere in erfindungsgemäßer Weise umsetzen. Man erhält auf diese Weise Gemische der beiden Enantiomere der gewünschten Zielverbindung.

Besonders bevorzugt eignet sich das erfindungsgemäße Verfahren zur Herstellung von optisch aktivem Citronellal der Formel (VI) 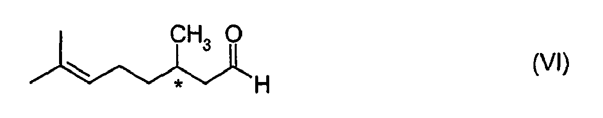 durch asymmetrische Hydrierung von Neral der Formel (VII) oder Geranial der Formel (VIII)

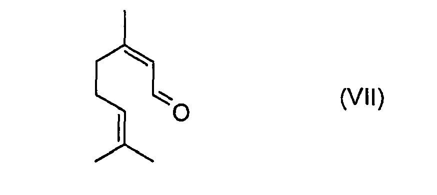

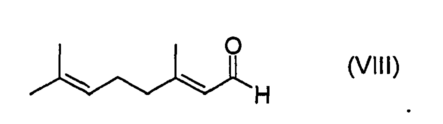

Auch Gemische von Geranial und Neral lassen sich in erfindungsgemäßer Weise umsetzen, wobei man, wie vorstehend beschrieben Gemische von D- bzw. L-Citronellal enthält, die optisch aktiv sind, falls die beiden Enantiomere nicht zu gleichen Teilen darin vorliegen.

Im Rahmen des erfindungsgemäßen Verfahrens insbesondere bevorzugt ist die erfindungsgemäße Herstellung D-Citronellal durch asymmetrische Hydrierung von Neral bzw. Geranial.

Das erfindungsgemäße Herstellverfahren wird in Gegenwart eines im Reaktionsgemisch löslichen, optisch aktiven Übergangsmetall-Katalysators, der mindestens einen Kohlenmonoxid-Liganden aufweist, durchgeführt.

Derartige Katalysatoren sind beispielsweise erhältlich durch Reaktion mindestens einer geeigneten, im Reaktionsgemisch löslichen Übergangsmetall-Verbindung mit einem optisch aktiven Liganden, der mindestens ein Phosphor- und/oder Arsenatom aufweist.

Bevorzugte Übergangsmetall-Verbindungen sind solche der Metalle der VIII. Nebengruppe des Periodensystems der Elemente, insbesondere Ru, Rh, Pd, Ir und Pt. Erfindungsgemäß besonders bevorzugte Übergangsmetalle der VIII. Nebengruppe des Periodensystems sind Rh und Ir.

Geeignete Verbindungen der genannten Übergangsmetalle sind insbesondere solche, die im gewählten Reaktionsmedium löslich sind, wie beispielsweise Salze oder Komplexverbindungen mit geeigneten Liganden wie z.B. Carbonyl, Acetylacetonat, Hydroxy, Cyclooctadien, Norbornadien, Cycloocten, Methoxy, Acetyl oder sonstige aliphatische oder aromatische Carboxylate. Im Rahmen des erfindungsgemäßen Verfahrens bevorzugte Übergangsmetallverbindungen sind Rh(I)- und Rh(III)- sowie Rh(0)-Verbindungen, Ir(I)-, Ir(III), Ir(IV)- sowie Ir(O)-Verbindungen, Ru(II), Ru(III), Ru(IV)-sowie Ru(0)-verbindungen, Pd(II)-, Pd(IV)- sowie Pd(O)-Verbindungen und Pt(II)-, Pt(IV)-sowie Pt(0)-Verbindungen. Bevorzugt sind solche Übergangsmetall-Verbindungen, die bereits mindestens einen CO-Liganden aufweisen. Daneben lassen sich auch Übergangsmetall-Verbindungen, die keinen CO-Liganden aufweisen, im Rahmen des erfindungsgemäßen Verfahrens als Ausgangsverbindung zur Herstellung der erfindungsgemäß einzusetzenden Katalysatoren verwenden. Diese werden unter den Bedingungen der erfindungsgemäß wahlweise durchzuführenden Präformierung bzw. der erfindungsgemäßen Hydrierbedingungen unter Zusatz von Kohlenmonoxid in die gewünschten Katalysatoren überführt.

Beispiele für erfindungsgemäß einsetzbare Übergangsmetall-Verbindungen sind: RhCl₃, Rh(OAc)₃, [Rh(cod)Cl]₂, Rh(CO)₂acac, [Rh(cod)OH]₂, [Rh(cod)OMe]₂, Rh₄(CO)₁₂, Rh₆(CO)₁₆ bzw. Ir₄(CO)₁₂, [Ir(cod)Cl]₂, wobei "acac" für einen Acetylacetonat- und "cod" für einen Cyclooctadien-Liganden steht.

Die genannten und weitere geeignete Übergangsmetallverbindungen und -komplexe sind bekannt und in der Literatur hinreichend beschrieben oder können vom Fachmann analog zu den bereits bekannten Verbindungen hergestellt werden.

Die genannten Übergangsmetallverbindungen setzt man erfindungsgemäß üblicherweise in einer Menge von etwa 0,01 bis etwa 1 mol-%, bevorzugt von etwa 0,05 bis etwa 0,5 mol-%, insbesondere von etwa 0,02 bis etwa 0,2 mol-% (bezogen auf die enthaltenen Übergangsmetallatome) im Verhältnis zur Menge an zu hydrierendem Substrat ein.

Bei unter kontinuierlichen Bedingungen durchgeführten Umsetzungen wählt man das Verhältnis von eingesetzter Menge an Übergangsmetallverbindung als Vorläufer des erfindungsgemäßen homogenen Katalysators zur Menge an zu hydrierendem Substrat vorteilhaft so, dass eine Katalysatorkonzentration im Bereich von etwa 100 ppm bis 10000 ppm, insbesondere im Bereich von etwa 200 ppm bis 5000 ppm eingehalten wird.

Die genannten Übergangsmetallverbindungen werden erfindungsgemäß mit einer weiteren Verbindung in Kontakt gebracht, die optisch aktiv, bevorzugt im wesentlichen enantiomerenrein ist (d.h. einen Enantiomerenüberschuss von mindestens etwa 99% aufweist) und mindestens ein Phosphor und/oder Arsenatom, bevorzugt mindestens ein Phosphoratom aufweist. Diese als chiraler Ligand zu bezeichnende Verbindung bildet im Reaktionsgemisch bzw. im Präformierungsgemisch mit der eingesetzten Übergangsmetall-Verbindung den erfindungsgemäß einzusetzenden Übergangsmetall-Katalysator.

Insbesondere bevorzugt sind solche chiralen Liganden die zwei Phosphoratome aufweisen und mit dem eingesetzten Übergangsmetall Chelatkomplexe bilden.

Als chirale Liganden eignen sich im Rahmen der vorliegenden Erfindung solche Verbindungen, wie sie beispielsweise in: I. Ojima (Hrsg.), Catalytic Asymmetric Synthesis, Wiley-VCh, 2. Auflage, 2000 oder in E. N. Jacobsen, A. Pfaltz, H. Yamamoto (Hrsg.), Comprehensive Asymmetric Catalysis, 2000, Springer oder in W. Tang, X. Zhang, Chem. Rev. 2003, 103, 3029-3069 beschrieben sind.

Beispielhaft seien die folgenden Verbindungen als erfindungsgemäß bevorzugt einsetzbare chirale Liganden angeführt:

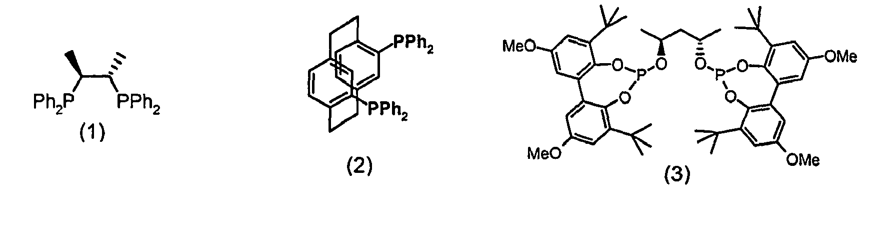

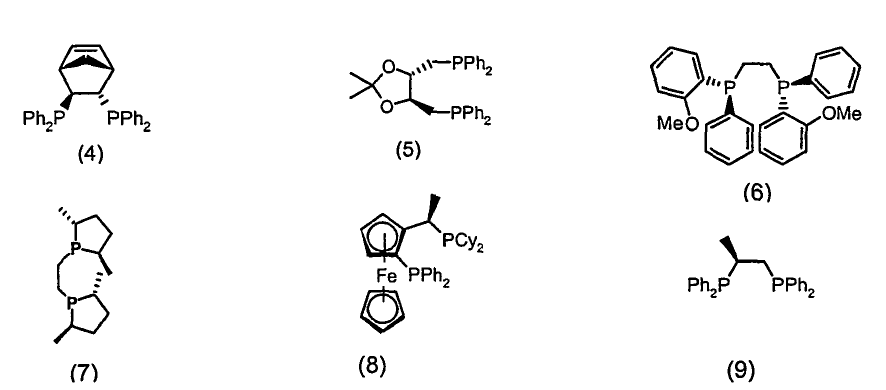

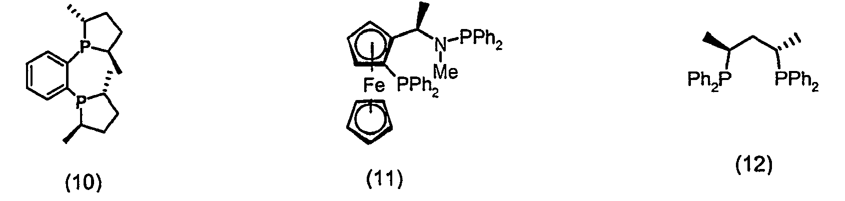

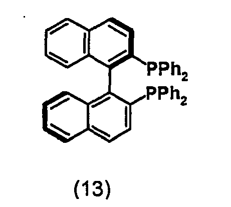

Weiterhin seien als erfindungsgemäß einsetzbare chirale Liganden beispielhaft die folgenden Verbindungen angeführt:

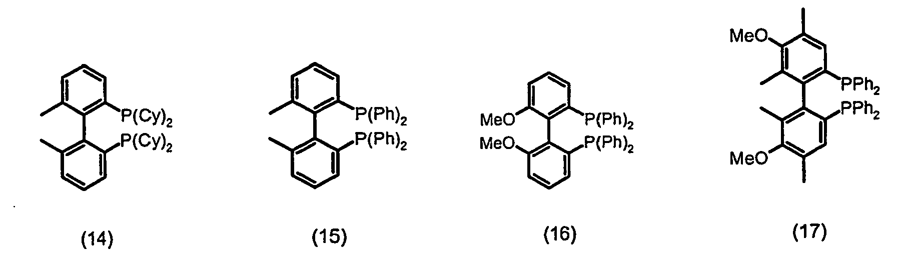

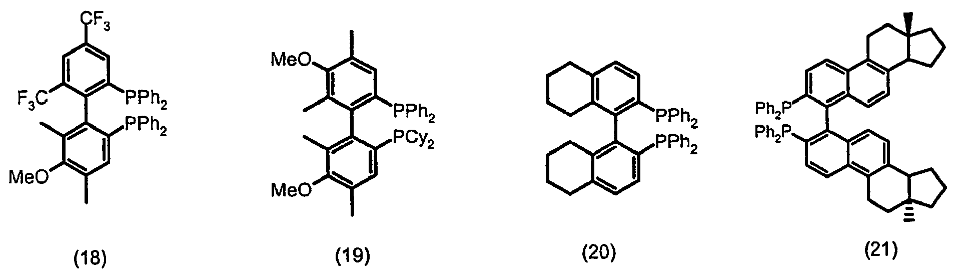

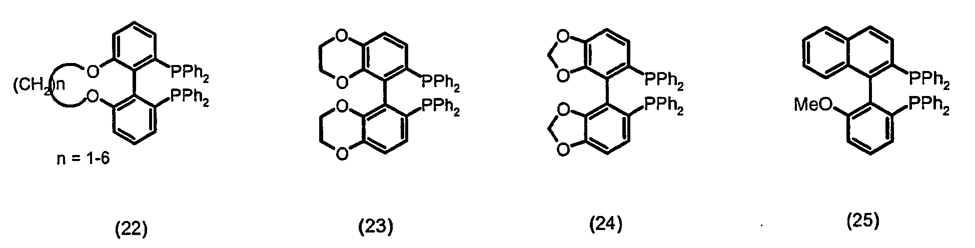

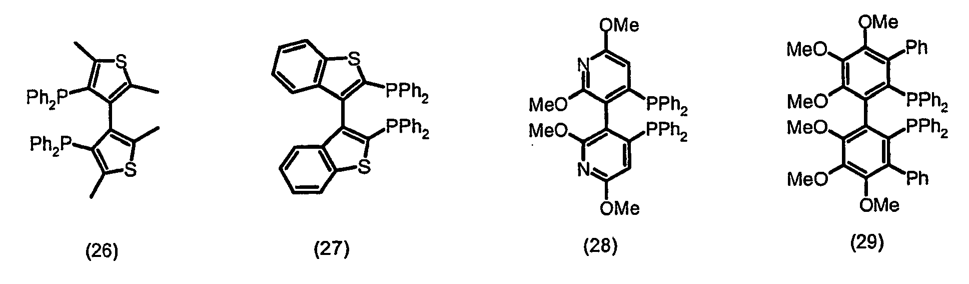

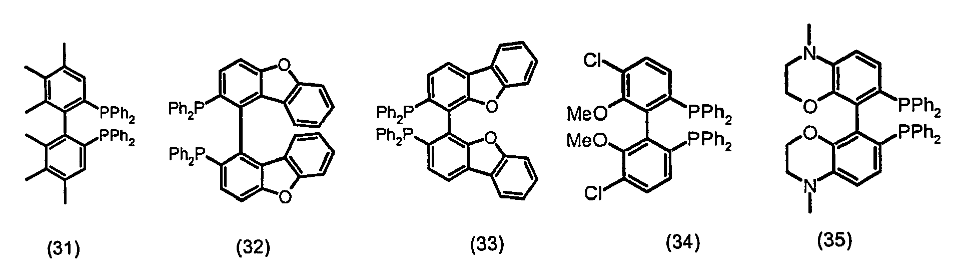

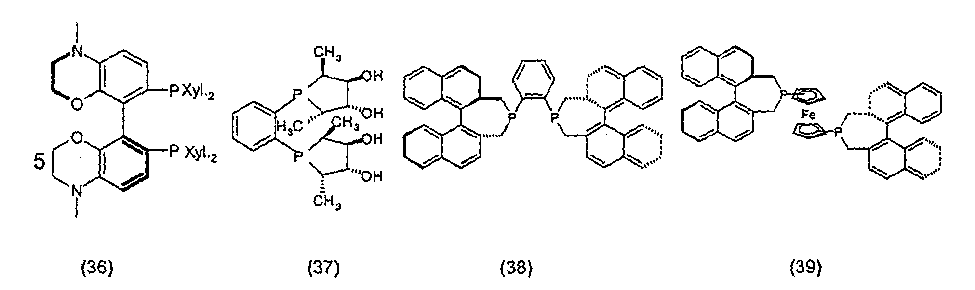

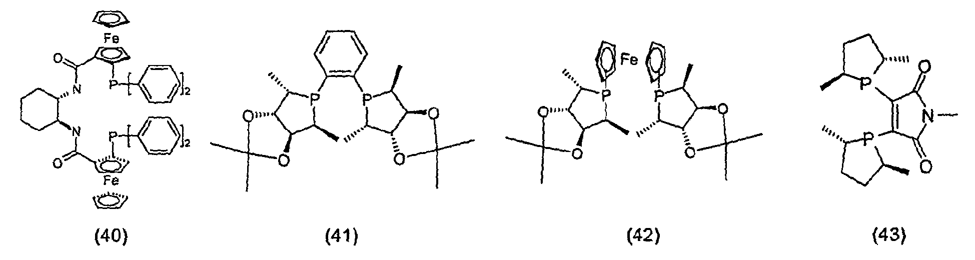

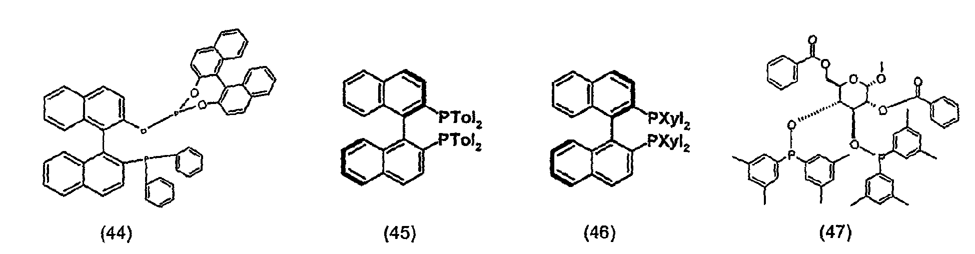

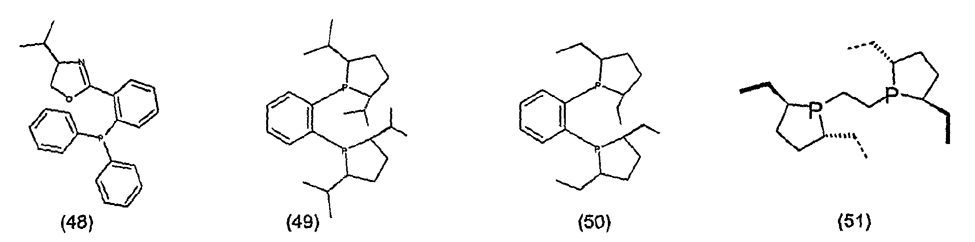

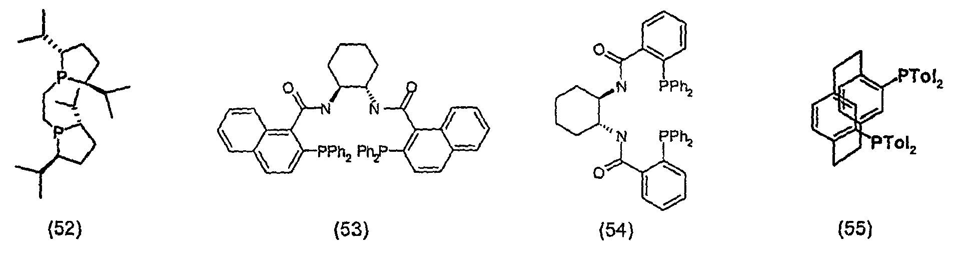

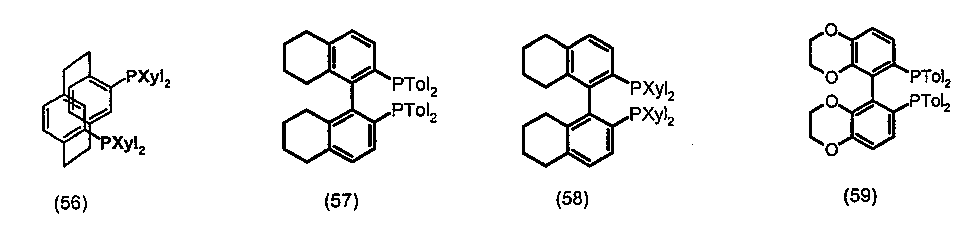

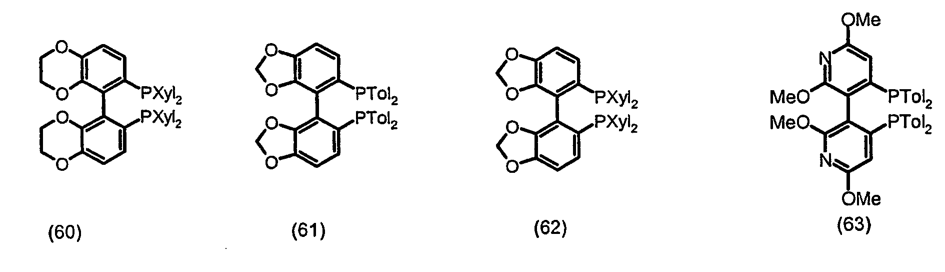

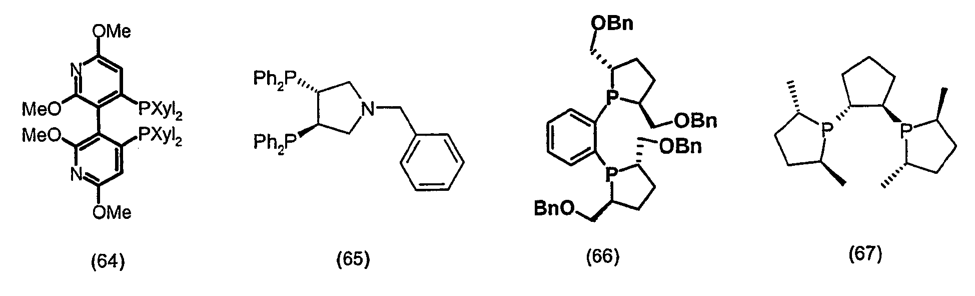

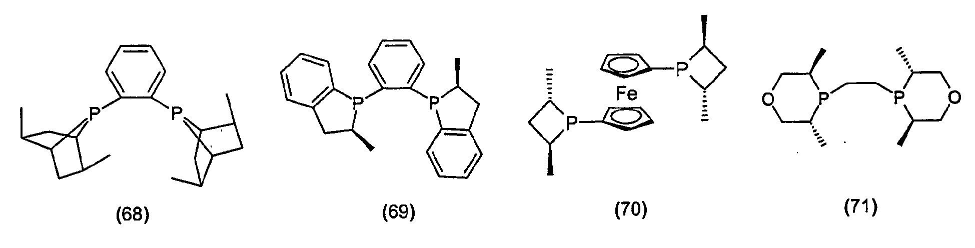

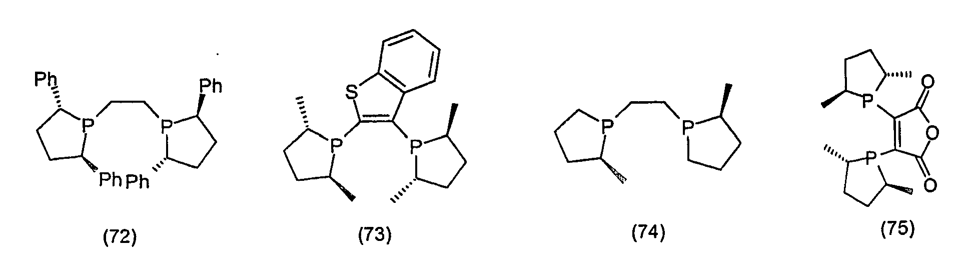

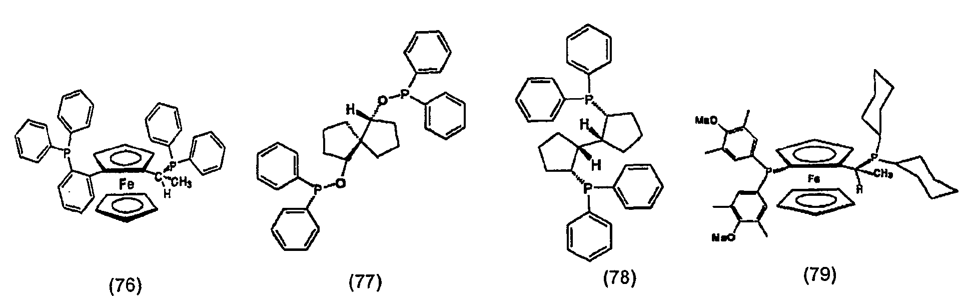

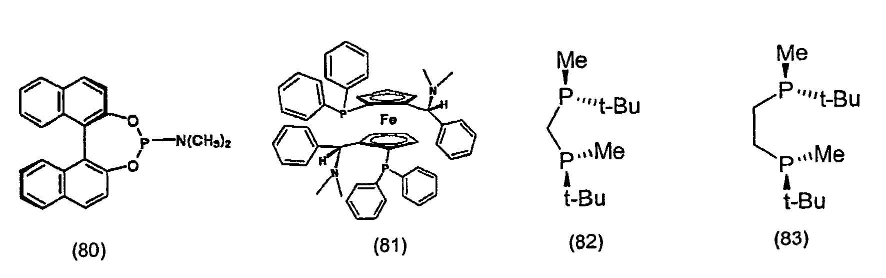

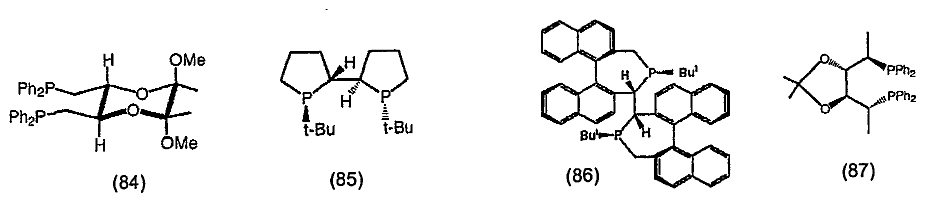

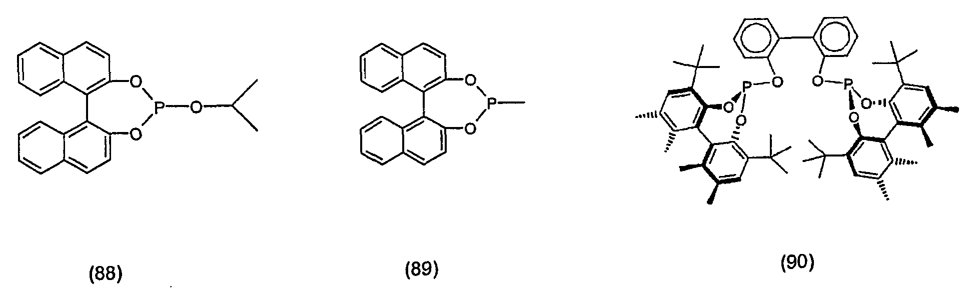

Dabei ist unter "Ph" Phenyl, "Cy" Cyclohexyl, "Xyl" Xylyl, "Tol" p-Tolyl und "Bn" Benzyl zu verstehen.

Erfindungsgemäß besonders bevorzugte Liganden sind die der Strukturformeln (1) bis (13) sowie (37), (38), (41), (43), (49), (50), (51), (52), (65), (66), (67), (68), (69), (71), (72), (73), (74), (75), (83), (84), (85), (86), (87).

Insbesondere bevorzugte Liganden sind solche der allgemeinen Formeln (IX) bis (XI) 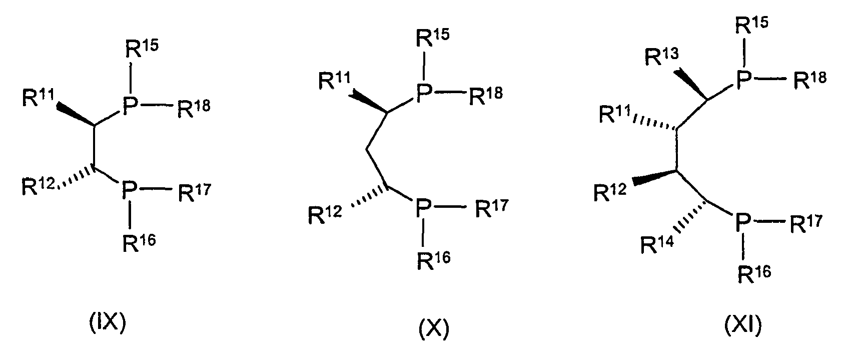 in denen
- R¹¹, R¹²:: jeweils unabhängig voneinander für einen unverzweigten, verzweig- ten oder cyclischen Alkylrest mit 1 bis 20 Kohlenstoffatomen, der gegebenenfalls eine oder mehrere, in der Regel 1 bis etwa 4, ethy- lenische Doppelbindungen und/oder einen oder mehrere, in der Re- gel 1 bis etwa 4, gleiche oder verschiedene Substituenten ausge- wählt aus der Gruppe der Substituenten OR¹⁹, NR²⁰R²¹, Halogen, C₆-C₁₀-Aryl und C₃-C₉-Hetaryl tragen kann und R¹¹ und R¹² gemein- sam einen 4 bis 20-gliedrigen Ring bilden können, der eines oder mehrere, in der Regel 1 oder 2 O-Atome beinhalten kann, stehen und
- R¹³, R¹⁴:: jeweils unabhängig voneinander Wasserstoff oder geradkettiges oder verzweigtes C₁- bis C₄-Alkyl bedeuten und
- R¹⁵, R¹⁶, R¹⁷, R¹⁸:: jeweils für C₆- bis C₁₀-Aryl, das gegebenenfalls einen oder mehrere, in der Regel 1 bis 8, bevorzugt 1 bis 4 Substituenten ausgewählt aus der Gruppe der Substtuenten C₁- bis C₄-Alkyl, C₆- bis C₁₀-Aryl, C₁- bis C₄-Alkoxy und Amino tragen kann, stehen und
- R¹⁹, R²⁰, R²¹:: jeweils unabhängig voneinander Wasserstoff, C₁-C₄-Alkyl, C₆-C₁₀- Aryl, C₇-C₁₂-Aralkyl oder C₇-C₁₂-Alkylaryl bedeuten, wobei
- R²⁰, R²¹:: gemeinsam auch eine Alkylenkette mit 2 bis 5 Kohlenstoffatomen, die durch N oder O unterbrochen sein kann, bedeuten können.

Im Rahmen des erfindungsgemäßen Verfahrens insbesondere bevorzugte Liganden sind solche der allgemeinen Formel (IX), insbesondere die im folgenden als "Chiraphos" bezeichnete Verbindungen der Formel (1).

Die gewählten chiralen Liganden können erfindungsgemäß jeweils in Form ihrer beiden Enantiomeren eingesetzt werden.

Bei Einsatz von chiralen Liganden mit zwei Phosphoratomen setzt man diese vorteilhaft in einer Menge von etwa 1 bis etwa 10 mol, bevorzugt etwa 1 bis etwa 4 mol pro mol an eingesetzter Übergangsmetall-Verbindung ein.

Aus der gewählten Übergangsmetall-Verbindung und dem gewählten chiralen Liganden können die eigentlichen, mindestens einen Kohlenmonoxid-Liganden enthaltenden Präkatalysatoren durch Zusammenbringen und ggf. Präformierung mit einer Mischung aus Wasserstoff und Kohlenmonoxid erhalten werden.

Das erfindungsgemäße Verfahren ist so durchzuführen, dass man den im Reaktionsgemisch löslichen, d.h. homogenen Katalysator, gewünschtenfalls in Anwesenheit des asymmetrisch zu hydrierenden Substrats, entweder vor der asymmetrischen Hydrierung mit einem Gasgemisch vorbehandelt, das Kohlenmonoxid und Wasserstoff enthält (d.h. eine sogenannte Präformierung des Katalysators durchführt) oder die asymmetrische Hydrierung in Gegenwart von dem Reaktionsgemisch zusätzlich zugeführtem Kohlenmonoxid durchführt oder eine Präformierung durchführt und anschließend die asymmetrische Hydrierung in Gegenwart von dem Reaktionsgemisch zusätzlich zugeführtem Kohlenmonoxid durchführt.

Bevorzugt führt man das erfindungsgemäße Verfahren so durch, dass man den Katalysator mit einem Kohlenmonoxid und Wasserstoff enthaltendem Gasgemisch vorbehandelt (d.h. präformiert) und die asymmetrische Hydrierung in Gegenwart von dem Reaktionsgemisch zusätzlich zugeführtem Kohlenmonoxid durchführt.

Führt man eine Präformierung durch, löst man üblicherweise die gewählte Übergangsmetall-Verbindung und den gewählten chiralen Liganden und gewünschtenfalls das asymmetrisch zu hydrierende Substrat in einem geeigneten, unter den reaktionsbedingungen inerten Lösungsmittel bzw. Lösungsmedium wie beispielsweise Ether, Tetrahydrofuran, Toluol, Chlorbenzol, Octadecanol, Biphenylether, Texanol, Marlotherm, Oxoöl 9N (Hydroformylierungsprodukte aus isomeren Octenen, BASF Aktiengesellschaft) und dergleichen mehr. Als Lösungsmedium können auch das umzusetzende Substrat, das Produkt oder bei der Umsetzung eventuell anfallende hochsiedende Nebenprodukte dienen. Der resultierenden Lösung wird, vorteilhafterweise in einem geeigneten Druckreaktor bzw. Autoklaven, bei einem Druck von üblicherweise etwa 5 bis etwa 350 bar, bevorzugt von etwa 20 bis etwa 200 bar und besonders bevorzugt von etwa 50 bis etwa 100 bar ein Gasgemisch aufgepresst, das Wasserstoff und Kohlenmonoxid enthält. Bevorzugt setzt man zur Präformierung ein Gasgemisch ein, das etwa
30 bis 99 Vol.-% Wasserstoff,
1 bis 70 Vol.-% Kohlenmonoxid und
0 bis 5 Vol.-% weiterer Gase enthält,
wobei sich die Angaben in Vol.-% zu 100 Vol.-% addieren müssen, enthält.

Besonders bevorzugt setzt man zur Präformierung ein Gasgemisch ein, das etwa
40 bis 80 Vol.-% Wasserstoff,
20 bis 60 Vol.-% Kohlenmonoxid und
0 bis 5 Vol.-% weiterer Gase enthält,
wobei sich die Angaben in Vol.-% zu 100 Vol.-% addieren müssen, enthält.

Ein zur Präformierung insbesondere bevorzugtes Gasgemisch ist sogenanntes Synthesegas, das üblicherweise zu etwa 35 bis 55 Vol-% aus Kohlenmonoxid neben Wasserstoff und Spuren weiterer Gase besteht.

Die erfindungsgemäße Präformierung des Katalysators wird üblicherweise bei Temperaturen von etwa 25°C bis etwa 100°C, bevorzugt bei etwa 40°C bis etwa 80°C durchgeführt. Führt man die Präformierung in Gegenwart des asymmetrisch zu hydrierenden Substrats durch, wählt man die Temperatur mit Vorteil so, dass es nicht in störendem Ausmaß zu einer Isomerisierung der zu hydrierenden Doppelbindung kommt. Die Präformierung ist üblicherweise nach etwa 1 h bis etwa 24 h, oft nach etwa 1 bis etwa 12 h abgeschlossen.

Im Anschluss an die optional durchzuführende Präformierung führt man erfindungsgemäß die asymmetrische Hydrierung des gewählten Substrats durch. Nach vorangegangener Präformierung lässt sich das gewählte Substrat in der Regel mit gutem Erfolg mit oder ohne Zuführung von zusätzlichem Kohlenmonoxid durchführen. Wurde auf eine vorangegangene Präformierung verzichtet, ist die erfindungsgemäße asymmetrische Hydrierung in Gegenwart von dem Reaktionssystem zusätzlich zugeführtem Kohlenmonoxid durchzuführen. Besonders vorteilhaft führt man eine Präformierung wie beschrieben durch und setzt dem Reaktionsgemisch während der asymmetrischen Hydrierung zusätzliches Kohlenmonoxid zu.

Die Zugabe von zusätzlichem Kohlenmonoxid kann auf verschiedene Weise vorgenommen werden: So kann das Kohlenmonoxid beispielsweise dem zur asymmetrischen Hydrierung eingesetzten Wasserstoff beigemischt werden oder auch direkt gasförmig in die Reaktionslösung eindosiert werden. Eine weitere Möglichkeit besteht beispielsweise darin, dem Reaktionsgemisch Verbindungen zuzusetzen, die leicht Kohlenmonoxid freisetzen, wie beispielsweise Formiate oder Oxalyl-Verbindungen.

Bevorzugt setzt man dem zur asymmetrischen Hydrierung eingesetzten Wasserstoff Kohlenmonoxid zu. Der Anteil an Kohlenmonoxid im eingesetzten Wasserstoff beträgt üblicherweise etwa 100 bis etwa 10000 ppm, bevorzugt etwa 500 bis etwa 5000 ppm und besonders bevorzugt etwa 600 bis etwa 3000 ppm.

Die erfindungsgemäße asymmetrische Hydrierung nimmt man vorteilhaft bei einem Druck von etwa 1 bis etwa 300 bar, bevorzugt von etwa 10 bis etwa 100 bar, insbesondere bei etwa 50 bis etwa 100 bar und einer Temperatur von in der Regel etwa 0°C bis etwa 100°C, bevorzugt etwa 0°C bis etwa 30°C, insbesondere bei etwa 10°C bis etwa 30°C vor.

Die Wahl des zur Durchführung der erfindungsgemäßen asymmetrischen Hydrierung einzusetzenden Lösemittels ist nicht kritisch. Geeignete Lösemittel sind beispielsweise jene zur Durchführung der erfindungsgemäßen Präformierung genannten. Mit besonderem Vorteil führt man die asymmetrische Hydrierung im gleichen Lösemittel durch wie die gegebenenfalls zuvor durchgeführte Präformierung.

Das erfindungsgemäße Verfahren kann mit gutem Erfolg mit und ohne Zugabe von tertiären Aminen durchgeführt werden. Bevorzugt führt man das erfindungsgemäße Verfahren in Abwesenheit, d.h. ohne Zugabe von zusätzlichen tertiären Aminen oder in Anwesenheit nur katalytischer Mengen von zusätzlichen tertiären Aminen durch.

Als Reaktionsgefäße zur Durchführung der erfindungsgemäßen asymmetrischen Hydrierung sind prinzipiell all jene geeignet, die Umsetzungen unter den genannten Bedingungen, insbesondere Druck und Temperatur, erlauben und für Hydrierreaktionen geeignet sind wie Beispielsweise Autoklaven, Rohrreaktoren, Blasensäulen, etc.

Mit Vorteil bricht man die Reaktion ab, wenn die Zielverbindung in der gewünschten Ausbeute und der gewünschte optischen Aktivität, d.h. mit dem gewünschten Enantiomerenüberschuss (ee) im Reaktionsgemisch vorliegt, wie es der Fachmann durch Routineuntersuchungen beispielsweise mittels chromatographischer Methoden feststellen kann. Üblicherweise ist die Hydrierung nach etwa 1 bis etwa 150 h, oft nach etwa 2 bis etwa 24 h abgeschlossen.

Durch das erfindungsgemäße Verfahren gelingt es, optisch aktive Carbonylverbindungen, insbesondere optisch aktive Aldehyde in hohen Ausbeuten und Enantiomerenüberschüssen bereitzustellen. Üblicherweise erhält man die gewünschten asymmetrisch hydrierten Verbindungen in einem Enantiomerenüberschuss von mindestens 80 % ee, oft mit einem Enantiomerenüberschuss von etwa 85 bis etwa 99 % ee. Dabei ist zu beachten, dass der maximal erzielbare Enantiomerenüberschuss von der Reinheit des eingesetzten Substrats, insbesondere im Hinblick auf die Isomerenreinheit der zu hydrierenden Doppelbindung, abhängen kann.

Demzufolge eignen sich als Ausgangssubstanzen insbesondere solche, die ein Isomerenverhältnis von mindestens etwa 90:10, bevorzugt mindestens etwa 95:5 bezüglich der E/Z-Doppelbindungsisomere aufweisen.

Das erfindungsgemäße Verfahren zeichnet sich dadurch aus, dass die eingesetzten homogenen Katalysatoren durch das zusätzlich ins Reaktionssystem eingebrachte Kohlenmonoxid stabilisiert werden, wodurch zum einen die Standzeit der Katalysatoren deutlich erhöht wird und zum anderen die Wiederverwendbarkeit der homogenen Katalysatoren ermöglicht wird.

So lässt sich beispielsweise das erhaltene Reaktionsprodukt durch dem Fachmann an sich bekannte Verfahren, wie z.B. durch Destillation, aus dem Reaktionsgemisch entfernen und der zurückbleibende Katalysator, gegebenenfalls nach abermaliger Präformierung, im Rahmen weiterer Umsetzungen nutzen.

Das erfindungsgemäße Verfahren kann demgemäss sowohl diskontinuierlich bzw. semikontinuierlich als auch kontinuierlich betrieben werden und eignet sich insbesondere für Umsetzungen in technischem Maßstab.

Im Rahmen einer besonders bevorzugten Ausführungsform des erfindungsgemäßen Verfahrens setzt man Neral oder Geranial, das bis zu etwa 5 mol-%, bevorzugt bis zu etwa 2 mol-% des jeweiligen Doppelbindungsisomeren enthält, zu optisch aktivem Citronellal um. Zur Bildung des Katalysators setzt man bevorzugt eine im Reaktionsgemisch lösliche Verbindung des Rhodium, insbesondere Rh(OAc)₃, [Rh(cod)Cl]₂, Rh(CO)₂acac, [Rh(cod)OH]₂, [Rh(cod)OMe]₂, Rh₄(CO)₁₂ oder Rh₆(CO)₁₆ und als chiralen Liganden (R,R)-Chiraphos bzw. (S,S)-Chiraphos ((2R, 3R)-(+)-2,3-bis(Diphenylphosphino)butan bzw. (2S, 3S)-(-)-2,3-bis(Diphenylphosphino)butan) im molaren Verhältnis von etwa 1:1 bis etwa 1:4 ein. In einer insbesondere bevorzugten Ausführungsform des erfindungsgemäßen Verfahren setzt man Neral, das bis zu etwa 5 mol-%, bevorzugt bis zu etwa 2 mol-% Geranial enthält in Gegenwart von Rh(OAc)₃, [Rh(cod)Cl]₂, Rh(CO)₂acac, [Rh(cod)OH]₂, [Rh(cod)OMe]₂, Rh₄(CO)₁₂ oder Rh₆(CO)₁₆ und (R,R)-Chiraphos zum D-Citronellal um. Bevorzugt präformiert man den Katalysator unter den vorstehend genannten Bedingungen und führt anschließend die asymmetrische Hydrierung in Gegenwart von Wasserstoff durch, der etwa 600 bis etwa 3000 ppm Kohlenmonoxid enthält. Im Rahmen der bevorzugten Ausführungsform verzichtet man vorteilhaft auf den Zusatz von Lösemitteln und führt die genannten Umsetzungen im umzusetzenden Substrat bzw. dem Produkt und gegebenenfalls in hochsiedenden Nebenprodukten als Lösemedium durch. Insbesondere bevorzugt ist die kontinuierliche Reaktionsführung unter Wiederverwendung bzw. Rückführung des erfindungsgemäß stabilisierten homogenen Katalysators.

Ein weiterer Aspekt der vorliegenden Erfindung betrifft ein Verfahren zur Herstellung von optisch aktivem Menthol unter Verwendung von nach dem erfindungsgemäßen Verfahren hergestelltem optisch aktivem Citronellal. Die Herstellung von optisch aktivem Menthol ausgehend von optisch aktivem Citronellal ist bekannt. Ein Schlüsselschritt ist hierbei die Cyclisierung von optisch aktivem Citronellal zu optisch aktivem Isopulegol wie beispielsweise in der EP-A 1 225 163 beschrieben.

Das erfindungsgemäß hergestellte optisch aktive Citronellal lässt sich, wie nachstehend schematisch für die Herstellung von L-Menthol der Formel (XIII) dargestellt, in Gegenwart einer geeigneten Säure, insbesondere einer Lewis-Säure zum L-Isopulegol der Formel (XII) cyclisieren und anschließend zum L-Menthol hydrieren.

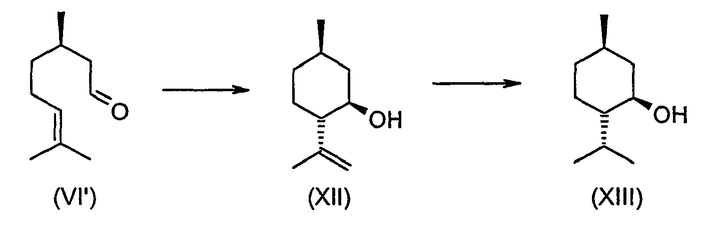

Ein weiterer Aspekt der vorliegenden Erfindung betrifft demgemäss die Verwendung von nach dem erfindungsgemäßen Verfahren hergestelltem optisch aktivem Citronellal zur Herstellung von optisch aktivem Menthol. Insbesondere betrifft die Erfindung die Verwendung von nach dem erfindungsgemäßen Verfahren hergestelltem D-Citronellal zur Herstellung von optisch aktivem L-Menthol.

Die folgenden Beispiele dienen der Verdeutlichung der Erfindung, ohne sie in irgend einer Weise zu beeinträchtigen:

### Beispiel 1: Asymmetrische Hydrierung von cis-Citral in Gegenwart von Kohlenmonoxid

17,9 mg Rh(CO)₂acac und 38,5 mg (R,R)-Chiraphos wurden unter Schutzgasatmosphäre in 20 g Toluol gelöst und in einen 100 ml Autoklaven überführt, der zuvor 3 mal mit einem Gemisch aus Kohlenmonoxid und Wasserstoff (1:1, Vol./Vol.) gespült wurde. Es wurde bei einem Druck von 8 bar CO/H₂1:1 und 60°C für 3 h gerührt und anschließend auf Raumtemperatur abgekühlt. Mittels einer Druckschleuse wurden dann 10,94 g Neral (Verhältnis der Doppelbindungsisomere Neral/Geranial = 99,1:0,9; Verhältnis Substrat/Katalysator = 1000) mit 15 bar H₂ zugepresst. Der Reaktionsdruck wurde durch Zupressen von Wasserstoff auf 80 bar eingestellt. Zur Reduktion des CO-Partialdrucks wurde der Druck drei mal und nach 3 h ein weiteres mal auf 8 bar erniedrigt und mittels Wasserstoff auf 80 bar nachgepresst. Nach 18 h wurde gaschromatographisch ein Umsatz von 99,9% und eine Ausbeute an D-Citronellal von 99,8% mit einer optischen Reinheit von 90% ee bestimmt.

### Beispiel 2: Asymmetrische Hydrierung von Neral in Gegenwart von Kohlenmonoxid

17,0 mg Rh(CO)₂acac und 43,8 mg (R,R)-Chiraphos wurden in 0,8 ml THF gelöst und in einem Autoklaven bei 80 bar Synthesegas (H₂/CO = 1:1, Vol./Vol.) und 60°C für 8 h gerührt. Anschließend wurden 39,00 g Neral (Verhältnis der Doppelbindungsisomere Neral/Geranial = 95,2:4,8; Verhältnis Substrat/Katalysator = 4000) gelöst und zusammen mit der Katalysatorlösung in einen 100 ml Autoklaven gegeben, der zuvor 3 mal mit CO/H₂ 1:1 (Vol./Vol.) gespült wurde. Der Reaktionsdruck wurde durch Zupressen von Wasserstoffgas, das 1000 ppm Kohlenmonoxid enthielt auf 80 bar eingestellt. Nach 144 h wurde gaschromatographisch ein Umsatz von 84,3% und eine Ausbeute von 80,9% an D-Citronellal mit einer optischen Reinheit von 64% ee bestimmt.

### Beispiel 3: Asymmetrische Hydrierung von Neral unter Wiederverwendung des Katalysators

23,7 mg Rh(CO)₂acac und 55,7 mg (R,R)-Chiraphos wurden unter Schutzgasatmosphäre in 24 g THF gelöst und in einen 100 ml Autoklaven gegeben, der zuvor 3 mal mit CO/H₂ 1:1 (Vol./Vol.) gespült wurde. Es wurde bei einem Druck von 80 bar CO/H₂ 1:1 und 60°C 3 h gerührt. Anschließend wurde auf Raumtemperatur abgekühlt und auf einen Druck von 8 bar CO/H₂ 1:1 entspannt. Mittels einer Druckschleuse wurden 13,2 g) Neral (Verhältnis der Doppelbindungsisomere Neral/Geranial = 99,4:0,6) mit 15 bar H₂ zugepresst. Der Reaktionsdruck wurde durch Zupressen von Wasserstoff auf 80 bar eingestellt. Zur Reduktion des CO-Partialdrucks wurde der Druck 5 mal auf 8 bar erniedrigt und mit Wasserstoff auf 80 bar nachgepresst. Der gaschromatographisch bestimmte Gehalt an CO im Gasraum betrug 510 ppm. Nach jeweils 20 h und 40 h werden weitere 13,20 g bzw. 19,80 g Neral zugegeben. Nach 66 h wurde gaschromatographisch ein Umsatz von 75,8% und eine Ausbeute von 72,8% D-Citronellal mit einer optischen Reinheit von 87% ee bestimmt.

Die Gesamt-turn-over-number bezogen auf die Ausbeute an D-Citronellal betrug 1030.

### Beispiel 4: Asymmetrische Hydrierung von cis-Citral unter Abdestillieren des Produkts und Wiederverwendung des Katalysators

8,4 mg Rh(CO)₂acac und 21,6 mg (R,R)-Chiraphos wurden in 0,8 ml THF gelöst und in einem Autoklaven bei 80 bar Synthesegas (H₂/CO = 1:1, Vol./Vol.) und 60°C für 8 h gerührt. Danach wurden 9 g Neral (Verhältnis der Doppelbindungsisomere Neral/Geranial = 95,2:4,8) in den Autoklaven gefüllt. Der Reaktionsdruck wurde durch Zupressen von Wasserstoffgas, das 1000 ppm Kohlenmonoxid enthielt, auf 80 bar eingestellt. Nach 24 h war ein Umsatz von 99% erreicht, der ee des erhaltenen D-Citronellals betrug 83%.

Nach Abdestillieren des Produktes werden weitere 8,5g Neral (Verhältnis der Doppelbindungsisomere Neral/Geranial = 95,2:4,8) zugegeben und 48 h bei 80 bar Wasserstoffgas, das 1000 ppm Kohlenmonoxid enthielt, hydriert. Der Umsatz betrugt 36%, der ee des erhaltenen D-Citronellals betrug 54%.

Nach abermaligem Abdestillieren des Produktes werden weitere 6,8 g Neral (Verhältnis der Doppelbindungsisomere Neral/Geranial = 95,2:4,8) zugegeben und 72 h bei 80 bar Wasserstoffgas, das 1000 ppm Kohlenmonoxid enthielt, hydriert. Der Umsatz betrug 13%, der ee des erhaltene D-Citronellals betrug 30%.

Die Gesamt-turn-over-number bezogen auf die Ausbeute an D-Citronellal betrug 2312.

### Beispiel 5: Asymmetrische Hydrierung von Neral unter Präformierung, Entfernung des Produkts und Wiederverwendung des Katalysators

30 mg Rh(CO)₂acac und 75 mg (R,R)-Chiraphos wurden in 3 ml THF gelöst und in einem Autoklaven bei 60°C in Gegenwart von 80 bar Synthesegas (H₂/CO = 1:1, Vol./Vol) für 20 h gerührt. Anschließend wurden 37 g Neral (Verhältnis der Doppelbindungsisomere Neral/Geranial = 96,6:3,4) zugegeben und die Lösung in einen 100 ml Autoklaven gegeben, der zuvor 3 mal mit CO/H₂ 1:1 (Vol./Vol.) gespült wurde. Der Reaktionsdruck wurde durch Zupressen von Wasserstoffgas, das 1000 ppm Kohlenmonoxid enthält, auf 80 bar eingestellt. Nach 24 h war ein Umsatz von >99% erreicht; der ee des erhaltene D-Citronellals betrug 87%.

Nach Abdestillieren des Produktes wurde der Destillationsrückstand mit THF verdünnt und in einem Autoklaven bei 60°C in Gegenwart von Synthesegas (H₂/CO = 1:1) bei einem Druck von 80 bar für 20 h gerührt. Danach wurden weitere 32 g Neral (Verhältnis der Doppelbindungsisomere Neral/Geranial = 96,6:3,4) zugegeben und 24 h bei einem Druck von 80 bar Wasserstoffgas, das 1000 ppm Kohlenmonoxid enthielt, hydriert. Der Umsatz betrug >99%, der ee des erhaltenen D-Citronellals betrug 87%.

Nach abermaligem Abdestillieren des Produktes wurde der Destillationsrückstand mit THF verdünnt und in einem Autoklaven bei 60°C in Gegenwart von 80 bar Synthesegas (H₂/CO = 1:1) für 20 h gerührt. Danach wurden weitere 32,96 g Neral (Verhältnis der Doppelbindungsisomere Neral/Geranial = 96,6:3,4) zugegeben und 24 h bei einem Druck von 80 bar Wasserstoffgas, das 1000 ppm Kohlenmonoxid enthielt, hydriert. Der Umsatz betrugt 90%, die optische Reinheit des erhaltenen D-Citronellals betrug 88% ee.

Das Experiment wurde nochmals wiederholt unter Zugabe von 33 g Neral (Verhältnis der Doppelbindungsisomere Neral/Geranial = 96,6:3,4). Man erhielt bei einem Umsatz von 17% D-Citronellal mit einer optischen Reinheit von 89% ee.

Die Gesamt-turn-over-number bezogen auf die Gesamtausbeute an D-Citronellal betrug 4975.

### Beispiel 6: Kontinuierlich betriebene asymmetrische Hydrierung von Neral

In einer kontinuierlich betriebenen Laboranlage wurde eine Lösung von 2,13 g Rh(CO)₂acac und 6,00 g (R,R)-Chiraphos in 70 g THF und 60 g Oxoöl 9N (BASF Aktitengesellschaft), die zuvor 20 h bei 60°C und einem Druck von 80 bar CO/H₂ 1:1 (Vol./Vol.) gerührt worden war und 170 g Neral (Verhältnis der Doppelbindungsisomere Neral/Geranial ca. 95:5) eingefüllt, woraufhin die Gasmischung im Präformierreaktor der Anlage auf 10000 ppm Kohlenmonoxid in Wasserstoff (80 bar), die Temperatur auf 60°C eingestellt wurde. Im Hydrierreaktor wurde eine Gasmischung von 1000 ppm Kohlenmonoxid in Wasserstoff (80 bar) und eine Temperatur von 25°C eingestellt.

Der Zulauf von frischem Edukt wurde auf 6 g/h eingestellt. Eine produkthaltige Fraktion wurde im Vakuum kontinuierlich so abdestilliert, dass der Anlageninhalt nahezu konstant blieb. Es wurden innerhalb von 19 Tagen 6,01 mol (927,7 g) D-Citronellal erhalten. Die Gesamt-turn-over-number bezogen auf die Ausbeute an D-Citronellal betrug 10914.

### Beispiel 7: Asymmetrische Hydrierung von 3-Methylcyclopent-2-enon in Gegenwart von Kohlenmonoxid

0,3049 g Rh(CO)₂acac und 0,7767 g (R,R)-Chiraphos wurden unter Schutzgasatmosphäre in 15 g Tetrahydrofuran gelöst und in einen 100 ml Autoklaven überführt, der zuvor 3 mal mit einem Gemisch aus Kohlenmonoxid und Wasserstoff (1:1, Vol./Vol.) gespült wurde. Es wurde bei einem Druck von 8 bar CO/H₂ 1:1 und 60°C für 24 h gerührt und anschließend auf Raumtemperatur abgekühlt. Aus der resultierenden Stamm-Lösung wurde unter Schutzgasatmosphäre 2,48 g entnommen und in 35 ml Tetrahydrofuran gelöst. Mittels einer Spritze wurden 2,0 g 3-Methylcyclopent-2-enon zugegeben und bei 50°C und 60 bar Wasserstoffgas, enthaltend 2000 ppm Kohlenmonoxid für 21 h gerührt. Der Umsatz zu 3-Methylcyclopentanon betrug 99 % der Enantiomerenüberschuss betrug 87 %.

### Vergleichsbeispiel 1: Asymmetrische Hydrierung von Neral

12,3 mg Rh₄(CO)₁₂ und 31,5 mg (S,S)-Chiraphos wurden unter Schutzgasatmosphäre in 15 g Toluol gelöst und in einen 100 ml Autoklaven überführt, der zuvor 3 mal mit H₂ gespült wurde. Es wurde bei 1,5 bar H₂ für 1,5 h gerührt, auf Normaldruck entspannt und 1 g Neral (Verhältnis der Doppelbindungsisomere Neral/Geranial = 98,7:1,3; Verhältnis Substrat/Katalysator = 100) gelöst in 15 g Toluol mittels einer Spritze zugegeben. Der Reaktionsdruck wurde durch Aufpressen von Wasserstoff auf 90 bar eingestellt. Gaschromatographische Reaktionskontrolle zeigte nach 15 h vollständigen Umsatz und eine gaschromatographisch bestimmte Ausbeute von 98% L-Citronellal mit einer optischen Reinheit von 96% ee.

## Patentansprüche

1. Verfahren zur Herstellung optisch aktiver Carbonylverbindungen durch asymmetrische Hydrierung α,β-ungesättigter Carbonylverbindungen in Gegenwart von im Reaktionsgemisch löslichen, optisch aktiven Übergangsmetall-Katalysatoren, die mindestens einen Kohlenmonoxid-Liganden aufweisen, **dadurch gekennzeichnet, dass** man den Katalysator mit einem Kohlenmonoxid und Wasserstoff enthaltenden Gasgemisch vorbehandelt und/oder die asymmetrische Hydrierung in Gegenwart von dem Reaktionsgemisch zusätzlich zugeführtem Kohlenmonoxid durchführt.

2. Verfahren nach Anspruch 1 zur Herstellung optisch aktiver Carbonylverbindungen der Formel (I) 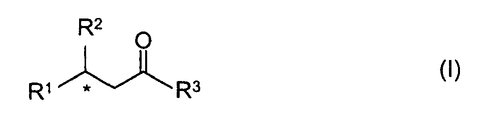 wobei die Reste
R¹, R² jeweils für einen unverzweigten, verzweigten oder cyclischen Alkyl- rest mit 1 bis 25 Kohlenstoffatomen, der gegebenenfalls eine oder mehrere ethylenische Doppelbindungen und/oder einen oder meh- rere gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann und der gemeinsam mit R³ einen 5 bis 25-gliedrigen Ring bilden kann, steht, mit der Maßgabe, dass R¹ und R² verschieden sind
R³ für Wasserstoff oder einen unverzweigten, verzweigten oder cycli- schen Alkylrest mit 1 bis 25 Kohlenstoffatomen, der gegebenenfalls eine oder mehrere ethylenische Doppelbindungen und/oder eine oder mehrere gleiche oder verschiedene Substituenten ausgewählt aus der Gruppe der Substituenten OR⁴, NR⁵R⁶, Halogen, C₆-C₁₀- Aryl und C₃-C₉-Hetaryl tragen kann, oder für steht OR⁷oder NR⁸R⁹ steht wobei
R⁴, R⁵, R⁶ jeweils unabhängig voneinander Wasserstoff C₁-C₆-Alkyl, C₆-C₁₀- Aryl, C₇-C₁₂-Aralkyl oder C₇-C₁₂-Alkylaryl bedeuten und
R⁵ und R⁶ gemeinsam auch eine Alkylenkette mit 2 bis 5 Kohlenstoffatomen, die durch N oder O unterbrochen sein kann, bedeuten können und
R⁷, R⁸ für einen unverzweigten, verzweigten oder cyclischen Alkylrest mit 1 bis 25 Kohlenstoffatomen, der gegebenenfalls eine oder mehrere ethylenische Doppelbindungen und eine oder mehrere gleiche oder verschiedene Substituenten ausgewählt aus der Gruppe der Sub- stituenten OR⁴, NR⁵R⁶, Halogen, C₆-C₁₀-Aryl und C₃-C₉-Hetaryl tra- gen kann, steht und gemeinsam mit R¹ oder R² einen 5 bis 25- gliedrigen Ring bilden können und
R⁹ für Wasserstoff, C₁-C₆-Alkyl, C₆-C₁₀-Aryl , C₇-C₁₂-Aralkyl oder C₇- C₁₂-Alkylaryl steht und
* ein asymmetrisches Kohlenstoffatom bezeichnet,
durch asymmetrische Hydrierung α,β-ungesättigter Aldehyde oder Ketone der Formel (II) 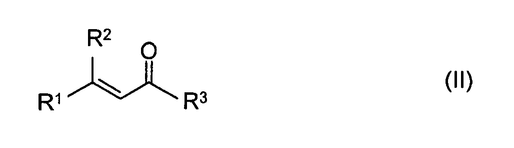 wobei die Reste R¹ bis R³ die oben angegebene Bedeutung besitzen.

3. Verfahren nach Anspruch 1 oder 2 zur Herstellung optisch aktiver Aldehyde oder Ketone durch asymmetrische Hydrierung α,β-ungesättigter Aldehyde oder Ketone.

4. Verfahren nach Anspruch 3 zur Herstellung optisch aktiver Aldehyde der Formel (III) 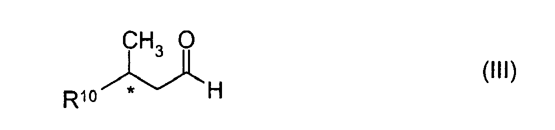 wobei
R¹⁰ für einen unverzweigten oder verzweigten Alkylrest mit 2 bis 25 Koh- lenstoffatomen, der gegebenenfalls 1 bis 5 ethylenische Doppelbindun- gen aufweisen kann, steht und
* ein asymmetrisches Kohlenstoffatom bezeichnet,
durch asymmetrische Hydrierung α,β-ungesättigter Aldehyde der Formel (IV) oder (V) 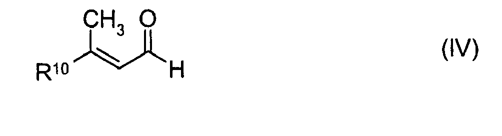 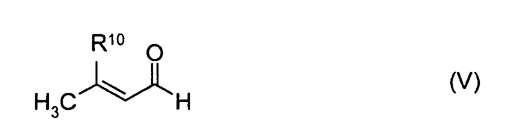 wobei der Rest R¹⁰ die oben angegebene Bedeutung besitzt.

5. Verfahren nach Anspruch 4 zur Herstellung von optisch aktivem Citronellal der Formel (V1) 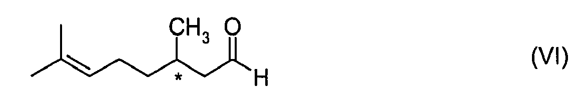 durch asymmetrische Hydrierung von Neral der Formel (VII) oder Geranial der Formel (VIII)

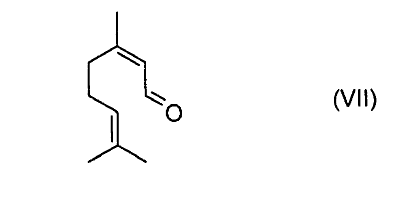

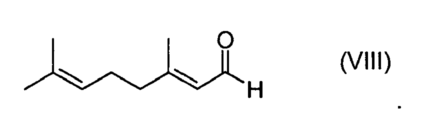

6. Verfahren nach Anspruch 5 zur Herstellung von D-Citronellal durch asymmetrische Hydrierung von Neral.

7. Verfahren nach einem der Ansprüche 1 bis 6, **dadurch gekennzeichnet, dass** man einen Übergangsmetall-Katalysator einsetzt, der erhältlich ist durch Reaktion mindestens einer im Reaktionsgemisch löslichen Übergangsmetall-Verbindung mit einem optisch aktiven Liganden der mindestens ein Phosphor- und/oder Arsenatom aufweist.

8. Verfahren nach Anspruch 7, **dadurch gekennzeichnet, dass** man als optisch aktiven Liganden eine Verbindung der allgemeinen Formeln (IX), (X) oder (XI) 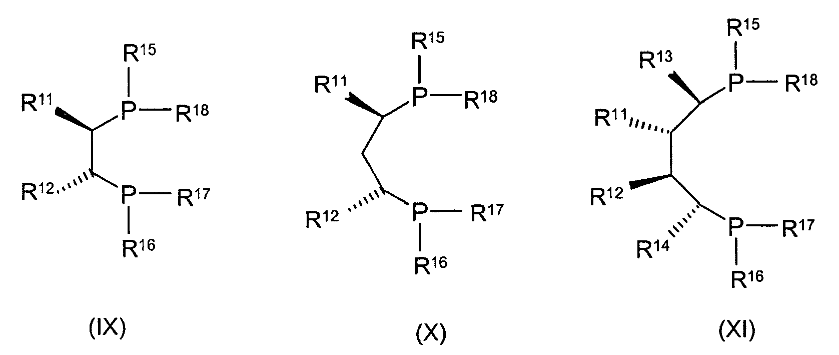 in denen
R¹¹, R¹²: jeweils unabhängig voneinander für einen unverzweigten, verzweigten oder cyclischen Alkylrest mit 1 bis 20 Kohlen- stoffatomen, der gegebenenfalls eine oder mehrere, in der Regel 1 bis etwa 4, ethylenische Doppelbindungen und/oder
R¹³, R¹⁴: einen oder mehrere, in der Regel 1 bis etwa 4, gleiche oder verschiedene Substituenten ausgewählt aus der Gruppe der Substituenten OR¹⁹, NR²⁰R²¹, Halogen, C₆-C₁₀Aryl und C₃- C₉-Hetaryl tragen kann und R¹¹ und R¹² gemeinsam einen 4 bis 20-gliedrigen Ring bilden können, der eines oder mehre- re, in der Regel 1 oder 2 O-Atome beinhalten kann, stehen und jeweils unabhängig voneinander Wasserstoff oder geradket- tiges oder verzweigtes C₁- bis C₄-Alkyl bedeuten und
R¹⁵, R¹⁶, R¹⁷, R¹⁸: jeweils für C₆- bis ^{C10}-Aryl, das gegebenenfalls einen oder mehrere, in der Regel 1 bis 8, bevorzugt 1 bis 4 Substituen- ten ausgewählt aus der Gruppe der Substtuenten C₁- bis C₄- Alkyl, C₆- bis C₁₀-Aryl, C₁- bis C₄-Alkoxy und Amino tragen kann, stehen und
R¹⁹, R²⁰, R²¹: jeweils unabhängig voneinander Wasserstoff, C₁-C₄-Alkyl, C₆-C₁₀-Aryl, C₇-C₁₂-Aralkyl oder C₇-C₁₂-Alkylaryl bedeuten, wobei
R²⁰, R²¹: gemeinsam auch eine Alkylenkette mit 2 bis 5 Kohlenstoff- atomen, die durch N oder O unterbrochen sein kann, bedeu- ten können

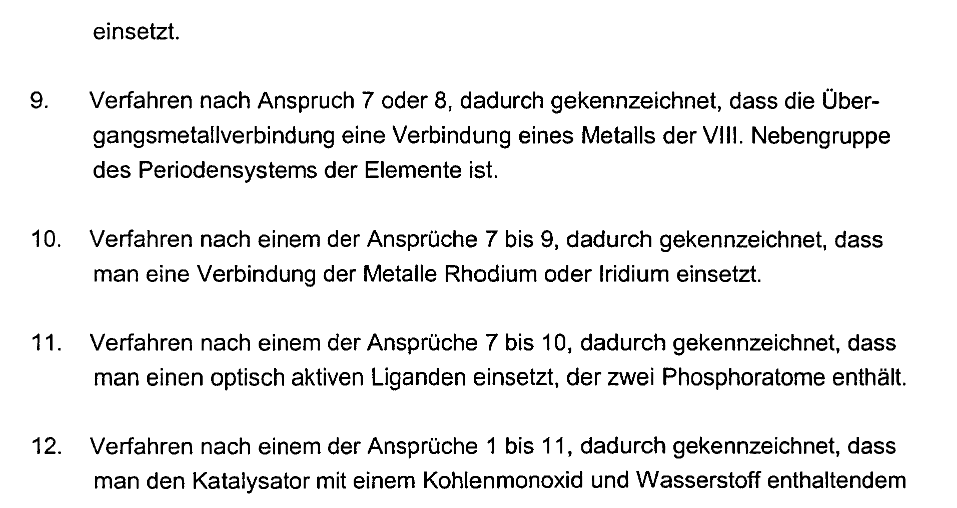

9. Verfahren nach Anspruch 7 oder 8, **dadurch gekennzeichnet, dass** die Übergangsmetallverbindung eine Verbindung eines Metalls der VII. Nebengruppe des Periodensystems der Elemente ist.

10. Verfahren nach Anspruch 7 bis 9, **dadurch gekennzeichnet, dass** man eine Verbindung der Metalle Rhodium oder Iridium einsetzt.

11. Verfahren nach Anspruch 7 bis 10, **dadurch gekennzeichnet, dass** man einen optisch aktiven Liganden einsetzt, der zwei Phosphoratome enthält.

12. Verfahren nach Anspruch 7 bis 11, **dadurch gekennzeichnet, dass** man den Katalysator mit einem Kohlenmonoxid und Wasserstoff enthaltendem Gasgemisch vorbehandelt und die asymmetrische Hydrierung in Gegenwart von dem Reaktionsgemisch zusätzlich zugeführtem Kohlenmonoxid durchführt.

13. Verfahren nach einem der Ansprüche 1 bis 12, **dadurch gekennzeichnet, dass** man den Katalysator mit einem Gasgemisch vorbehandelt, das
30 bis 99 Vol.-% Wasserstoff,
1 bis 70 Vol.-% Kohlenmonoxid und
0 bis 5 Vol.-% weiterer Gase enthält,
wobei sich die Angaben in Vol.-% zu 100 Vol.-% addieren müssen.

14. Verfahren nach einem der Ansprüche 1 bis 13, **dadurch gekennzeichnet, dass** man zur asymmetrischen Hydrierung Wasserstoff einsetzt, der 100 bis 10.000 ppm Kohlenmonoxid enthält.

15. Verfahren nach einem der Ansprüche 1 bis 14, **dadurch gekennzeichnet, dass** man die asymmetrische Hydrierung bei einem Druck von 10 bis 100 bar durchführt.

16. Verfahren nach einem der Ansprüche 1 bis 15, **dadurch gekennzeichnet, dass** man es kontinuierlich durchführt.

17. Verfahren zur Herstellung von optisch aktivem Menthol unter Verwendung von nach einem der Ansprüche 1 bis 16 hergestelltem optisch aktivem Citronellal.

18. Verfahren nach Anspruch 17, **dadurch gekennzeichnet, dass** man nach einem der Ansprüche 1 bis 15 hergestelltes optisch aktives Citronellal in Gegenwart einer Lewis-Säure zu L-Isopulegol cyclisiert und anschließend hydriert.

19. Verwendung von nach einem der Ansprüche 1 bis 16 hergestelltem optisch aktivem Citronellal zur Herstellung von optisch aktivem Menthol.

20. Verfahren zur Herstellung von optisch aktivem Menthol umfassend die Schritte
a. Herstellung von optisch aktivem Citronellal gemäß einen der Ansprüche 1 bis 16,
b) Cyclisierung des in Schritt a) erhaltenen optisch aktiven Citonellals zu optisch aktivem Isopulegol
und
c) Hydrierung des in Schritt b) erhaltenen optisch aktiven Isopulegols zu optisch aktivem Menthol.

## Claims

1. A process for preparing optically active carbonyl compounds by asymmetrically hydrogenating α,β-unsaturated carbonyl compounds in the presence of optically active transition metal catalysts which are soluble in the reaction mixture and have at least one carbon monoxide ligand, which comprises pretreating the catalyst with a gas mixture comprising carbon monoxide and hydrogen and/or carrying out the asymmetric hydrogenation in the presence of carbon monoxide supplied additionally to the reaction mixture.

2. The process according to claim 1 for preparing optically active carbonyl
compounds of the formula (I) 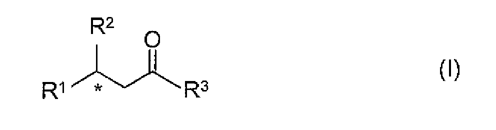 where
the R¹, R² radicals are each an unbranched, branched or cyclic alkyl radical which has from 1 to 25 carbon atoms and may optionally bear one or more ethylenic double bonds and/or one or more identical or different substituents selected from the group of the OR⁴, NR⁵R⁶, halogen, C₆-C₁₀-aryl and C₃-C₉-hetaryl
the R³ substituents and which, together with R³, may form a 5- to 25- membered ring, with the proviso that R¹ and R² are different, radical is hydrogen or an unbranched, branched or cyclic alkyl radical which has from 1 to 25 carbon atoms and may optionally bear one or more ethylenic double bonds and/or one or more identical or different substituents selected from the group of the OR⁴, NR⁵R⁶, halogen, C₆-C₁₀-aryl and C₃-C₉- hetaryl substituents, or is OR⁷ or NR⁸R⁹,
where
R⁴, R⁵, R⁶ are each independently hydrogen, C₁-C₆-alkyl, C₆-C₁₀-aryl, C₇- C₁₂-aralkyl or C₇-C₁₂-alkylaryl and
R⁵ and R⁶ together may also be an alkylene chain having from 2 to 5 carbon atoms which may be interrupted by N or O and
R⁷, R⁸ are each an unbranched, branched or cyclic alkyl radical which has from 1 to 25 carbon atoms and may optionally bear one or more ethylenic double bonds and one or more identical or different substituents selected from the group of the OR⁴, NR⁵R⁶, halogen, C₆-C₁₀-aryl and C₃-C₉-hetaryl substituents, and, together with R¹ or R², may form a 5- to 25-membered ring and
R⁹ is hydrogen, C₁-C₆-alkyl, C₆-C₁₀-aryl, C₇-C₁₂-aralkyl or C₇- C₁₂-alkylaryl and
* indicates an asymmetric carbon atom,
by asymmetrically hydrogenating α,β-unsaturated aldehydes or ketones of the formula (II) 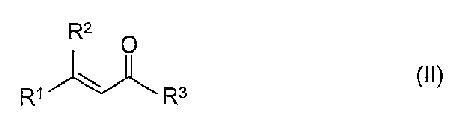 where the R¹ to R³ radicals are each as defined above.

3. The process according to claim 1 or 2 for preparing optically active aldehydes or ketones by asymmetrically hydrogenating α,β-unsaturated aldehydes or ketones.

4. The process according to claim 3 for preparing optically active aldehydes of
the formula (III) 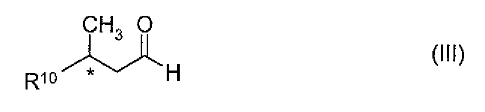 where
R¹⁰ is an unbranched or branched alkyl radical which has from 2 to 25 carbon atoms and may optionally have from 1 to 5 ethylenic double bonds and
* indicates an asymmetric carbon atom,
by asymmetrically hydrogenating α,β-unsaturated aldehydes of the formula (IV) or (V) 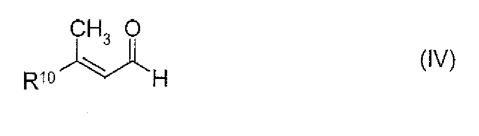 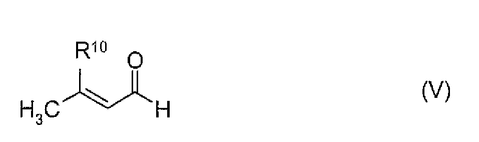 where the R¹⁰ radical is as defined above.

5. The process according to claim 4 for preparing optically active citronellal of the formula (VI) 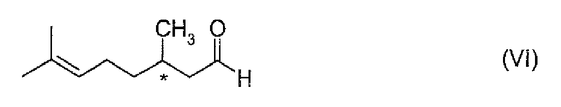 by asymmetrically hydrogenating neral of the formula (VII) or geranial of the formula (VIII)

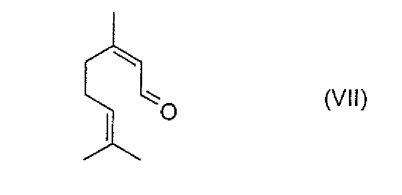

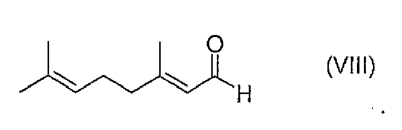

6. The process according to claim 5 for preparing D-citronellal by asymmetrically hydrogenating neral.

7. The process according to any of claims 1 to 6, wherein a transition metal catalyst is used which is obtainable by reaction of at least one transition metal compound soluble in the reaction mixture with an optically active ligand which has at least one phosphorus and/or arsenic atom.

8. The process according to claim 7, werein the optically active ligand used is of the general formulae (IX), (X) or (XI) 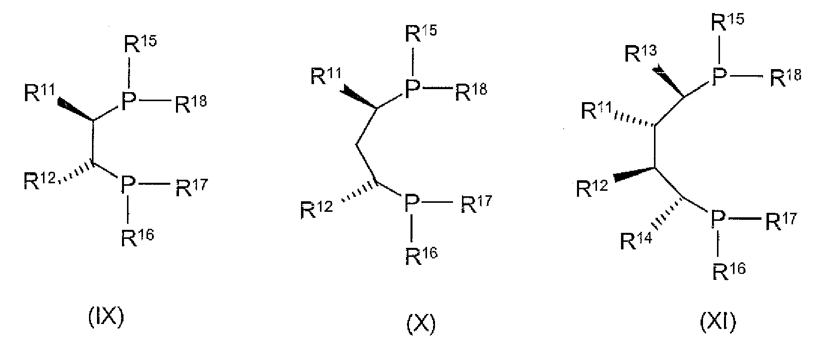 in which
R¹¹, R¹²: are each independently an unbranched, branched or cyclic alkyl radical which has from 1 to 20 carbon atoms and may optionally bear one or more, generally from 1 to about 4, ethylenic double bonds and/or one or more, generally from 1 to about 4, identical or different substituents selected from the group of the OR¹⁹, NR²⁰R²¹, halogen, C₆-C₁₀-aryl and C₃-C₉-hetaryl substituents, and R¹¹ and R¹² together may form a 4- to 20-membered ring which may contain one or more, generally 1 or 2, oxygen atoms, and
R¹³, R¹⁴: are each independently hydrogen or straight-chain or branched C₁- to C₄-alkyl, and
R¹⁵, R¹⁶, R¹⁷, R¹⁸: are each C₆- to C₁₀-aryl, each of which may optionally bear one or more, generally from 1 to 8, preferably from 1 to 4, substituents selected from the group of the C₁- to C₄-alkyl, C₆- to C₁₀-aryl, C₁- to C₄-alkoxy and amino substituents, and
R¹⁹, R²⁰, R²¹: are each independently hydrogen, C₁-C₄-alkyl, C₆-C₁₀- aryl, C₇-C₁₂-aralkyl or C₇-C₁₂-alkylaryl, where
R²⁰, R²¹; together may also be an alkylene chain which has from 2 to 5 carbon atoms and may be interrupted by N or O.

9. The process according to claim 7 or 8, wherein the transition metal compound is a compound of a metal of transition group VIII of the Periodic Table of the Elements.

10. The process according to any of claims 7 to 9, wherein a compound of the metals rhodium or iridium is used.

11. The process according to any of claims 7 to 10, wherein an optically active ligand is used which comprises two phosphorus atoms.

12. The process according to any of claims 1 to 11, wherein the catalyst is pretreated with a gas mixture comprising carbon monoxide and hydrogen, and the asymmetric hydrogenation is carried out in the presence of carbon monoxide supplied additionally to the reaction mixture.

13. The process according to any of claims 1 to 12, wherein the catalyst is pretreated with a gas mixture which comprises
from 30 to 99% by volume of hydrogen,
from 1 to 70% by volume of carbon monoxide and
from 0 to 5% by volume of further gases,
the data in % by volume having to add up to 100% by volume.

14. The process according to any of claims 1 to 13, wherein hydrogen which comprises from 100 to 10 000 ppm of carbon monoxide is used for the asymmetric hydrogenation.

15. The process according to any of claims 1 to 14, wherein the asymmetric hydrogenation is carried out at a pressure of from 10 to 100 bar.

16. The process according to any of claims 1 to 15, which is carried out continuously.

17. A process for preparing optically active menthol using optically active citronellal prepared according to any of claims 1 to 16.

18. The process according to claim 17, wherein optically active citronellal prepared according to any of claims 1 to 15 is cyclized in the presence of a Lewis acid to give L-isopulegol and subsequently hydrogenated.

19. The use of optically active citronellal prepared according to any of claims 1 to 16 for preparing optically active menthol.

20. A process for preparing optically active menthol, comprising the steps of
a) preparing optically active citronellal according to any of claims 1 to 16,
b) cyclizing the optically active citronellal obtained in step a) to give optically active isopulegol
and
c) hydrogenating the optically active isopulegol obtained in step b) to give optically active menthol.

## Revendications

1. Procédé pour la préparation de composés carbonyle optiquement actifs par hydrogénation asymétrique de composés carbonyle α,β-insaturés en présence de catalyseurs à base de métaux de transition solubles dans le mélange réactionnel, optiquement actifs, qui présentent au moins un ligand monoxyde de carbone, **caractérisé en ce qu'**on prétraite le catalyseur avec un mélange gazeux contenant du monoxyde de carbone et de l'hydrogène et/ou on réalise l'hydrogénation asymétrique en présence de monoxyde de carbone introduit en supplément dans le mélange réactionnel.

2. Procédé selon la revendication 1 pour la préparation de composés carbonyle optiquement actifs de formule (I), 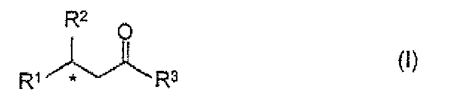 dans laquelle les radicaux
R¹, R² représentent à chaque fois un radical alkyle non ramifié, ramifié ou cyclique comprenant 1 à 25 atomes de carbone, qui peut porter, le cas échéant, une ou plusieurs doubles liaisons éthyléniques et/ou un ou plusieurs substituants identiques ou différents, choisis dans le groupe des substituants OR⁴, NR⁵R⁶, halogène, C₆-C₁₀-aryle et C₃-C₉-hétéroaryle et qui peut former, ensemble avec R³ un cycle de 5 à 25 chaînons, à condition que R¹ et R² soient différents
R³ représente hydrogène ou un radical alkyle non ramifié, ramifié ou cyclique comprenant 1 à 25 atomes de carbone, qui peut porter, le cas échéant, une ou plusieurs doubles liaisons éthyléniques et/ou un ou plusieurs substituants identiques ou différents, choisis dans le groupe des substituants OR⁴, NR⁵R⁶, halogène, C₆-C₁₀-aryle et C₃-C₉-hétéroaryle, ou représente OR⁷ ou NR⁸R⁹
où
R⁴, R⁵, R⁶ signifient, à chaque fois indépendamment l'un de l'autre, hydrogène, C₁-C₆-alkyle, C₆-C₁₀-aryle, C₇-C₁₂-aralkyle ou C₇-C₁₂-alkylaryle et
R⁵ et R⁶ peuvent également signifier, ensemble, une chaîne alkylène comprenant 2 à 5 atomes de carbone, qui peut être interrompue par N ou O et
R⁷, R⁸ représentent un radical alkyle non ramifié, ramifié ou cyclique comprenant 1 à 25 atomes de carbone, qui peut porter, le cas échéant, une ou plusieurs doubles liaisons éthyléniques et un ou plusieurs substituants identiques ou différents, choisis dans le groupe des substituants OR⁴, NR⁵R⁶, halogène, C₆-C₁₀-aryle et C₃-C₉-hétéroaryle et qui peut former, ensemble avec R¹ ou R² un cycle de 5 à 25 chaînons et
R⁹ représente hydrogène, C₁-C₆-alkyle, C₆-C₁₀-aryle, C₇-C₁₂-aralkyle ou C₇-C₁₂-alkylaryle et
* désigne un atome de carbone asymétrique,
par hydrogénation asymétrique d'aldéhydes ou de cétones α,β-insaturés de formule (II) 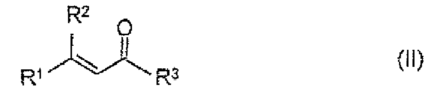 où les radicaux R¹ à R³ présentent la signification indiquée ci-dessus.

3. Procédé selon la revendication 1 ou 2 pour la préparation d'aldéhydes ou de cétones optiquement actifs par hydrogénation asymétrique d'aldéhydes ou de cétones α,β-insaturés.

4. Procédé selon la revendication 3 pour la préparation d'aldéhydes optiquement actifs de formule (III) 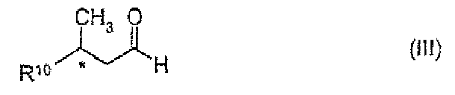 où
R¹⁰ représente un radical alkyle linéaire ou ramifié comprenant 2 à 25 atomes de carbone qui peut le cas échéant présenter 1 à 5 doubles liaisons éthyléniques et
* désigne un atome de carbone asymétrique,
par hydrogénation asymétrique d'aldéhydes α,β-insaturés de formule (IV) ou (V) 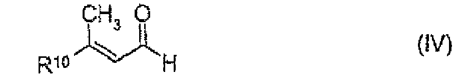 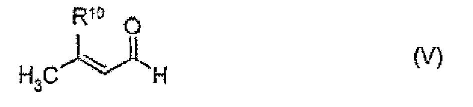 où le radical R¹⁰ présente la signification indiquée ci-dessus.

5. Procédé selon la revendication 4 pour la préparation de citronellal optiquement actif de formule (VI) 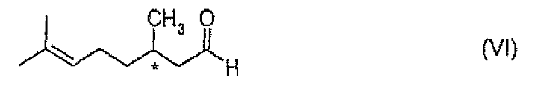 par hydrogénation asymétrique de néral de formule (VII) ou de géranial de formule (VIII)

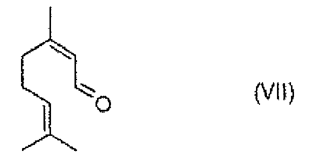

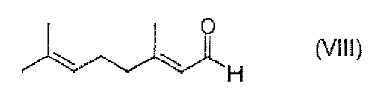

6. Procédé selon la revendication 5 pour la préparation de D-citronellal par hydrogénation asymétrique de néral.

7. Procédé selon l'une quelconque des revendications 1 à 6, **caractérisé en ce qu'**on utilise un catalyseur à base de métaux de transition qui peut être obtenu par réaction d'au moins un composé de métal de transition soluble dans le mélange réactionnel avec un ligand optiquement actif qui présente au moins un atome de phosphore et/ou d'arsenic.

8. Procédé selon la revendication 7, **caractérisé en ce qu'**on utilise comme ligands optiquement actifs un composé des formules générales (IX), (X) ou (XI) 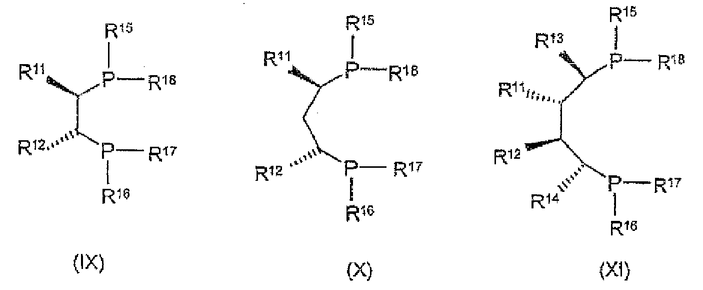 où
R¹¹, R¹² : représentent à chaque fois, indépendamment l'un de l'autre, un radical alkyle non ramifié, ramifié ou cyclique comprenant 1 à 20 atomes de carbone, qui peut porter, le cas échéant, une ou plusieurs, généralement 1 à environ 4, doubles liaisons éthyléniques et/ou un ou plusieurs, généralement 1 à environ 4, substituants identiques ou différents, choisis dans le groupe des substituants OR¹⁹, NR²⁰R²¹, halogène, C₆-C₁₀- aryle et C₃-C₉-hétéroaryle et R¹¹ et R¹² peuvent former ensemble un cycle de 4 à 20 chaînons, qui peut comporter un ou plusieurs, généralement 1 ou 2 atomes de O et
R¹³, R¹⁴ : représentent, à chaque fois indépendamment l'un de l'autre, hydrogène ou C₁-C₄-alkyle linéaire ou ramifié et
R¹⁵, R¹⁶, R¹⁷, R¹⁸ : représentent à chaque fois C₆- C₁₀-aryle, qui peut le cas échéant porter un ou plusieurs, généralement 1 à 8, de préférence 1 à 4 substituants choisis dans le groupe des substituants C₁-C₄-alkyle, C₆-C₁₀-aryle, C₁-C₄- alcoxy et amino, et
R¹⁹, R²⁰, R²¹ : signifient, à chaque fois indépendamment l'un de l'autre, hydrogène, C₁-C₄-alkyle, C₆-C₁₀- aryle, C₇-C₁₂-aralkyle ou C₇-C₁₂-alkylaryle, où
R²⁰, R²¹ : peuvent également signifier, ensemble, une chaîne alkylène comprenant 2 à 5 atomes de carbone, qui peut être interrompue par N ou O.

9. Procédé selon la revendication 7 ou 8, **caractérisé en ce que** le composé à base de métaux de transition est un composé d'un métal du VIIIème groupe secondaire du système périodique des éléments.

10. Procédé selon l'une quelconque des revendications 7 à 9, **caractérisé en ce qu'**on utilise un composé des métaux rhodium ou iridium.

11. Procédé selon l'une quelconque des revendications 7 à 10, **caractérisé en ce qu'**on utilise un ligand optiquement actif qui contient deux atomes de phosphore.

12. Procédé selon l'une quelconque des revendications 1 à 11, **caractérisé en ce qu'**on prétraite le catalyseur avec un mélange gazeux contenant du monoxyde de carbone et de l'hydrogène et on réalise l'hydrogénation asymétrique en présence de monoxyde de carbone introduit en supplément dans le mélange réactionnel.

13. Procédé selon l'une quelconque des revendications 1 à 12, **caractérisé en ce qu'**on prétraite le catalyseur avec un mélange gazeux qui contient
- 30 à 99% en volume d'hydrogène
- 1 à 70% en volume de monoxyde de carbone et
- 0 à 5% en volume d'autres gaz,
les indications en % en volume devant totaliser 100% en volume.

14. Procédé selon l'une quelconque des revendications 1 à 13, **caractérisé en ce qu'**on utilise pour l'hydrogénation asymétrique de l'hydrogène qui contient 100 à 10 000 ppm de monoxyde de carbone.

15. Procédé selon l'une quelconque des revendications 1 à 14, **caractérisé en ce qu'**on réalise l'hydrogénation asymétrique à une pression de 10 à 100 bars.

16. Procédé selon l'une quelconque des revendications 1 à 15, **caractérisé en ce qu'**on le réalise en continu.

17. Procédé pour la préparation de menthol optiquement actif en utilisant du citronellal optiquement actif préparé selon l'une quelconque des revendications 1 à 16.

18. Procédé selon la revendication 17, **caractérisé en ce qu'**on cyclise du citronellal optiquement actif préparé selon l'une quelconque des revendications 1 à 15 en présence d'un acide de Lewis en L-isopulégol puis il est hydrogéné.

19. Utilisation de citronellal optiquement actif préparé selon l'une quelconque des revendications 1 à 16 pour la préparation de menthol optiquement actif.

20. Procédé pour la préparation de menthol optiquement actif, comprenant les étapes
a. préparation de citronellal optiquement actif selon l'une quelconque des revendications 1 à 16,
b. cyclisation du citronellal optiquement actif obtenu dans l'étape a) en isopulégol optiquement actif et
c. hydrogénation de l'isopulégol optiquement actif obtenu dans l'étape b) en menthol optiquement actif.
